(19) Europäisches Patentamt
European Patent Office
Office européen des brevets

(11) EP 2 505 585 B1

(12) **EUROPEAN PATENT SPECIFICATION**

(45) Date of publication and mention of the grant of the patent:
**03.08.2016 Bulletin 2016/31**

(21) Application number: **09851686.7**

(22) Date of filing: **26.11.2009**

(51) Int Cl.:
***C07D 265/16*** *(2006.01)* ***C08K 5/357*** *(2006.01)*

(86) International application number:
**PCT/JP2009/070267**

(87) International publication number:
**WO 2011/064897 (03.06.2011 Gazette 2011/22)**

(54) **BIS-BENZOXAZINONE COMPOUND**

BIS-BENZOXAZINON-VERBINDUNG

COMPOSÉ BIS-BENZOXAZINONE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**03.10.2012 Bulletin 2012/40**

(60) Divisional application:
**16161415.1**

(73) Proprietor: **Teijin Chemicals, Ltd.**
**Chiyoda-ku**
**Tokyo 100-0013 (JP)**

(72) Inventors:
- **IBUSUKI Shun**
  **Tokyo 100-0013 (JP)**
- **KOGA Takashi**
  **Tokyo 100-0013 (JP)**
- **MIYAKE Akira**
  **Setouchi-shi**
  **Okayama 701-4212 (JP)**
- **HAYASHI Masayuki**
  **Setouchi-shi**
  **Okayama 701-4212 (JP)**
- **YOSHIDA Masaya**
  **Setouchi-shi**
  **Okayama 701-4212 (JP)**

(74) Representative: **Adam, Holger et al**
**Kraus & Weisert**
**Patentanwälte PartGmbB**
**Thomas-Wimmer-Ring 15**
**80539 München (DE)**

(56) References cited:
**EP-A1- 1 553 140**
**EP-A2- 1 314 755**
**WO-A1-02/064600**
**WO-A1-03/035735**
**WO-A1-03/095557**
**WO-A1-2009/041715**
**WO-A1-2009/123147**
**JP-A- 7 330 783**
**JP-A- 2004 526 707**
**JP-A- 2005 507 006**
**JP-A- 2006 124 600**
**JP-A- 2009 286 717**
**US-A1- 2005 215 750**
**US-A1- 2011 015 314**

EP 2 505 585 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Invention

The present invention relates to a production method of a bisbenzoxazinone compound.

Description of the Prior Art

Polycarbonate resin is used for various purposes as it has excellent transparency, heat resistance and mechanical strength. However, when the polycarbonate resin is used outdoors for a long time or used in a cover for lamp light sources, its weatherability may come into question. Therefore, it is proposed that an ultraviolet absorbent should be contained in the polycarbonate resin. However, according to the type of the ultraviolet absorbent added, the color and moist heat resistance of the polycarbonate resin may deteriorate.

There is known a bisbenzoxazinone compound as an ultraviolet absorbent for resins. The bisbenzoxazinone compound can be produced by reacting anthranilic acid with a dicarboxylic acid dihalide (Patent Documents 1 to 3). It can also be produced by reacting isatoic anhydride with a dicarboxylic acid dihalide (Patent Document 4).

In all the methods of Patent Documents 1 to 3 in which anthranilic acid is used as a raw material, an alkali metal compound such as sodium carbonate or sodium hydroxide is used as a dehydrohalogenation agent in the reaction. Therefore, a trace amount of a halogenated alkali metal salt such as sodium chloride is contained in the obtained bisbenzoxazinone compound. When this bisbenzoxazinone compound containing an alkali metal salt is added as an ultraviolet absorbent to the polycarbonate resin, the moist heat resistance of the polycarbonate resin is degraded by the alkali metal.

Meanwhile, Patent Document 4 proposes a method of producing a bisbenzoxazinone compound having a low yellowness index and a low content of sodium by reacting purified isatoic anhydride with a dicarboxylic acid dihalide in pyridine. However, this document does not teach concrete means of purification. The amount of the dicarboxylic acid dihalide is not specified, and the amount of the dicarboxylic acid dihalide in Example is excessive, thereby producing an impurity. In the method in which isatoic anhydride is used as a raw material, the isatoic anhydride is more expensive than anthranilic acid used in the methods in which it is used as a raw material. Therefore, a bisbenzoxazinone compound which is obtained from anthranilic acid and does not degrade the color and moist heat resistance of polycarbonate resin is desired.

(Patent Document 1) JP-A 58-194854
(Patent Document 2) JP-A 61-291575
(Patent Document 3) JP-A 2003-155468
(Patent Document 4) WO2003/035735

Summary of the Invention

It is an object of the present invention to provide a method of producing a bisbenzoxazinone compound from an anthranilic acid derivative without using an alkali metal compound such as sodium hydroxide or sodium carbonate as a dehydrohalogenation agent. The bisbenzoxazinone compound can provide excellent color, moist heat resistance and weatherability to a polycarbonate resin.

A resin composition which comprises an aromatic polycarbonate and has excellent color, moist heat resistance and weatherability and a molded product thereof can be prepared.

The inventors of the present invention have conducted intensive studies to solve the above problem and have found that, when a reaction between an anthranilic acid derivative (1) and an aromatic dicarboxylic acid dihalide (2), represented by the following reaction formula, is carried out in an organic solvent which does not participate in the reaction and an inert gas such as nitrogen is introduced into a reaction solution or a reactor to remove hydrogen halide (HX in the formula) generated by the reaction, the reaction proceeds smoothly, and an amide compound (3) is obtained as an intermediate at a high yield, and when the obtained amide compound is reacted with a dehydrating agent after that, a bisbenzoxazinone compound (4) having high purity can be produced without using an alkali metal compound. The present invention has been accomplished based on this finding.

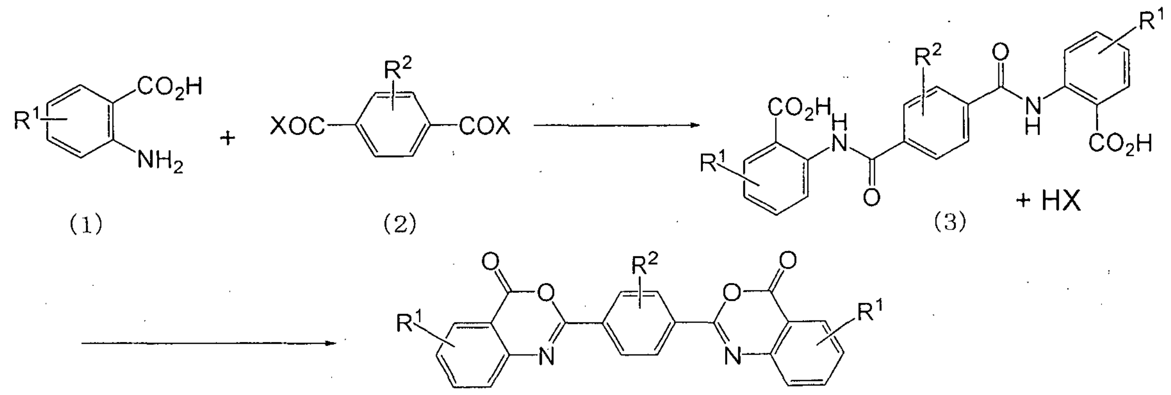

(1) (2) (3) (4)

That is, the present invention includes the following inventions.

1. A method of producing a bisbenzoxazinone compound represented by the following formula (4):

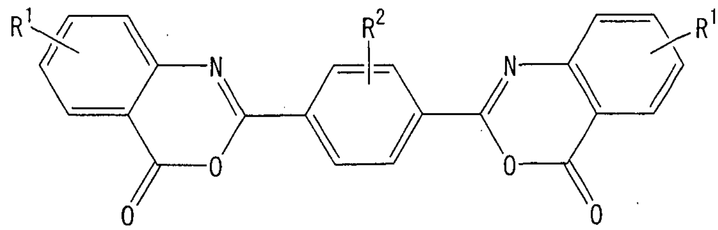

(4)

($R^1$ and $R^2$ are each independently a hydrogen atom, hydroxyl group, alkyl group having 1 to 3 carbon atoms, alkoxy group having 1 to 3 carbon atoms, acyl group having 1 to 3 carbon atoms, acyloxy group having 1 to 3 carbon atoms, alkoxycarbonyl group having 1 to 3 carbon atoms, halogen atom, nitro group or carboxyl group),

and having (i) a purity of 98 % or more,
(ii) a content of a compound represented by the following formula (7) of less than 0.15 %:

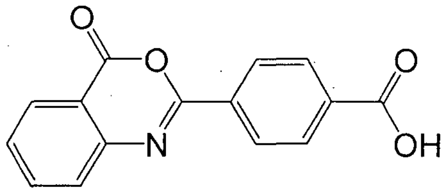

(7)

(iii) a maximum peak in the measurement of the light reflectance at a visible range of its powder at 380 to 480 nm and a maximum light reflectance of 100 to 120 %,
(iv) a metal sodium content of less than 50 ppm and
(v) a YI value of -10 to 10, comprising the steps of:

(Step 1) reacting an anthranilic acid derivative represented by the following formula (1) with an aromatic dicarboxylic acid dihalide represented by the following formula (2) in an organic solvent in an inert gas stream to produce an amide compound represented by the following formula (3), wherein the amount of the inert gas is 1 to 50 liters/hr based on 1 mole of the anthralinic acid derivative:

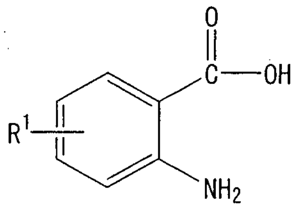

(1)

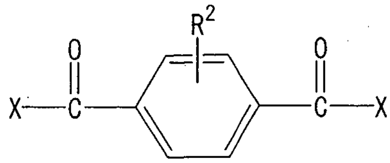

(2)

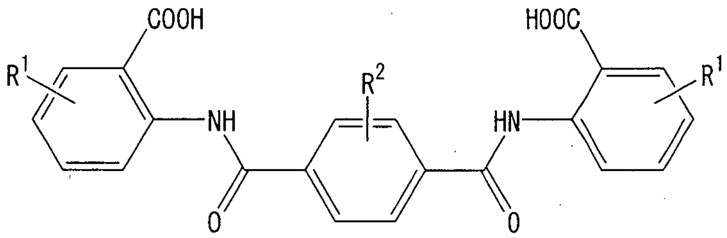

(3)

($R^1$ and $R^2$ are as defined above, and X is a halogen atom); and
(Step 2) reacting the obtained amide compound represented by the formula (3) with a dehydrating agent.

2. The production method according to the above paragraph 1, wherein the organic solvent is at least one selected from the group consisting of a ketone, an aromatic hydrocarbon, a halogenated hydrocarbon and an ether.
3. The production method according to the above paragraph 2, wherein the inert gas is nitrogen.
4. The production method according to the above paragraph 2, wherein the dehydrating agent is acetic anhydride.
5. The production method according to the above paragraph 2, wherein, in the formulas (1) to (4), $R^1$ is a hydrogen atom, $R^2$ is a hydrogen atom, and X is a chlorine atom.

Detailed Description of the Preferred Embodiment (bisbenzoxazinone compound)

The bisbenzoxazinone compound produced according to the present invention is represented by the following formula (4).

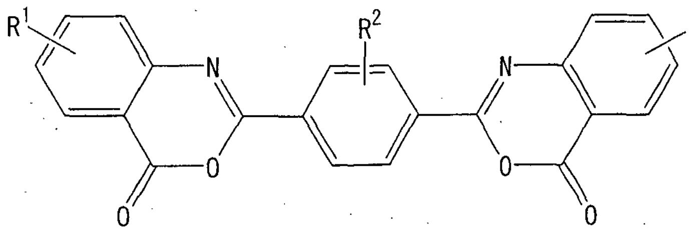

(4)

In the above formula, $R^1$ and $R^2$ are each independently a hydrogen atom, hydroxyl group, alkyl group having 1 to 3 carbon atoms, alkoxy group having 1 to 3 carbon atoms, acyl group having 1 to 3 carbon atoms, acyloxy group having 1 to 3 carbon atoms, alkoxycarbonyl group having 1 to 3 carbon atoms, halogen atom, nitro group or carboxyl group.
Examples of the alkyl group having 1 to 3 carbon atoms include methyl group, ethyl group and propyl group.

Examples of the alkoxy group having 1 to 3 carbon atoms include methoxy group, ethoxy group and propoxy group. Examples of the acyl group (R-CO-) having 1 to 3 carbon atoms include acetyl group and acryloyl group. Examples of the acyloxy group (R-CO-O-) having 1 to 3 carbon atoms include groups having a methyl group or an ethyl group as R. Examples of the alkoxycarbonyl group (-CO-OR) having 1 to 3 carbon atoms include groups having a methyl group or an ethyl group as R. Examples of the halogen atom include fluorine atom, chlorine atom and bromine atom. More preferably, $R^1$ is a hydrogen atom. A bisbenzoxazinone compound of the formula (4) in which $R^1$ is a hydrogen atom and $R^2$ is a hydrogen atom is preferred.

(purity)

The bisbenzoxazinone compound produced according to the present invention has a purity of 98 % or more, preferably 98.2 % or more, more preferably 98.5 % or more. The purity of the bisbenzoxazinone compound is measured by HPLC (high-speed liquid chromatography).

(compound represented by formula (7))

The bisbenzoxazinone compound produced according to the present invention has a content of a compound represented by the following formula (7) of less than 0.15 wt%, preferably less than 0.12 wt%, more preferably less than 0.1 wt%. The content of the compound represented by the formula (7) is measured by HPLC (high-speed liquid chromatography).

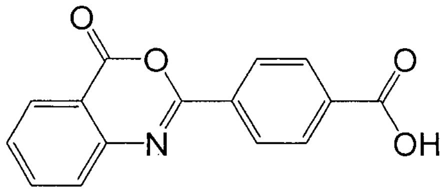

(7)

(measurement of light reflectance)

The maximum peak in the measurement of the light reflectance at a visible range of the bisbenzoxazinone compound in powdery form is existent at a range of 380 to 480 nm, preferably 400 to 460 nm, more preferably 440 to 460 nm.

The maximum light reflectance in the measurement of the light reflectance at a visible range of the bisbenzoxazinone compound in powdery form is 100 to 120 %, preferably 100 to 110 %.

The light reflectance is obtained by charging the powdery bisbenzoxazinone compound into a cylindrical glass container having a diameter of 30 mm and a height of 13 mm and measuring the maximum peak value at a range of 380 to 480 nm with the TR-1800MK-II of Tokyo Denshoku Co., Ltd. A reflection method (2 degree field of view, illuminant C) is used for the measurement.

(content of metal sodium)

The bisbenzoxazinone compound produced according to the present invention has a metal sodium content of less than 50 ppm, preferably less than 40 ppm, more preferably less than 25 ppm. The content of metal sodium is measured by flame atomic absorptiometry.

(YI value)

The bisbenzoxazinone compound produced according to the present invention has a YI value of -10 to 10, preferably -5 to 5, more preferably 0 to 3. The YI value of the powdery bisbenzoxazinone compound is measured with the TR-1800MK-II of Tokyo Denshoku Co., Ltd. The reflection method (2 degree field of view, illuminant C) is used for the measurement.

(method of producing bisbenzoxazinone compound)

The bisbenzoxazinone compound can be produced by the following method. The method comprises step 1 for obtaining an amide compound (3) and step 2 for obtaining a bisbenzoxazinone compound (4) by dehydrating the amide compound to cyclize it.

(step 1)

The step 1 is to obtain the amide compound (3) by reacting an anthranilic acid derivative (1) with an aromatic dicarboxylic acid dihalide (2) in an organic solvent in an inert gas stream.

The anthranilic acid derivative is represented by the following formula (1).

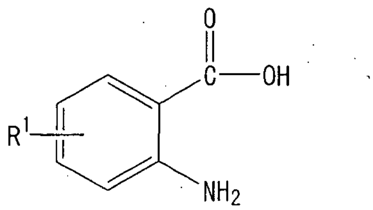

(1)

In the above formula, $R^1$ is a hydrogen atom, hydroxyl group, alkyl group having 1 to 3 carbon atoms, alkoxy group having 1 to 3 carbon atoms, acyl group having 1 to 3 carbon atoms, acyloxy group having 1 to 3 carbon atoms, alkoxycarbonyl group having 1 to 3 carbon atoms, halogen atom, nitro group or carboxyl group.

Examples of the alkyl group having 1 to 3 carbon atoms include methyl group, ethyl group and propyl group. Examples of the alkoxy group having 1 to 3 carbon atoms include methoxy group, ethoxy group and propoxy group. Examples of the acyl group (R-CO-) having 1 to 3 carbon atoms include acetyl group and acryloyl group. Examples of the acyloxy group (R--CO-O-) having 1 to 3 carbon atoms include groups having a methyl group or en ethyl group as R. Examples of the alkoxycarbonyl group (-CO-OR) having 1 to 3 carbon atoms include groups having a methyl group or an ethyl group as R. Examples of the halogen atom include fluorine atom, chlorine atom and bromine atom. More preferably, $R^1$ is a hydrogen atom. A specific example of the anthranilic acid derivative is anthranilic acid.

The aromatic dicarboxylic acid dihalide is represented by the following formula (2).

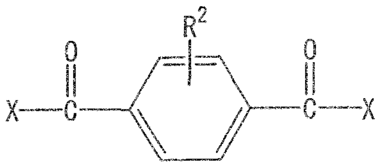

(2)

In the above formula, X is a halogen atom. Examples of the halogen atom include fluorine atom, chlorine atom, bromine atom and iodine atom. It is preferably a chlorine atom.

$R^2$ is a hydrogen atom, hydroxyl group, alkyl group having 1 to 3 carbon atoms, alkoxy group having 1 to 3 carbon atoms, acyl group having 1 to 3 carbon atoms, acyloxy group having 1 to 3 carbon atoms, alkoxycarbonyl group having 1 to 3 carbon atoms, halogen atom, nitro group or carboxyl group. Examples of these groups are the same as in the formula (1), More preferably, $R^2$ is a hydrogen atom. A specific example of the aromatic dicarboxylic acid dihalide is terephthalic acid dichloride.

In the above reaction, the compound (3) is obtained by reacting 2 moles of the anthranilic acid derivative with 1 mole of the aromatic dicarboxylic acid dihalide. To suppress the formation of a by-product represented by the following formula (5) when 1 mole of the anthranilic acid derivative is reacted with 1 mole of the aromatic dicarboxylic acid dihalide, it is preferred that the anthralinic acid derivative should be used in an amount slightly larger than its stoichiometric amount. That is, the amount of the aromatic dicarboxylic acid dihalide used in the reaction is preferably 0.43 to 0.5 mole, more preferably 0.4 7 to 0.5 mole based on 1 mole of the anthralinic acid derivative.

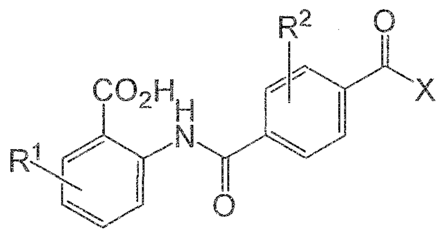

(5)

In the above formula, $R^1$ and $R^2$ are as defined in the above formulas (1) and (2), preferably hydrogen atoms.

A tertiary amine such as pyridine is used as an organic base in the reaction between the isatoic anhydride and the aromatic dicarboxylic acid dihalide as in Patent Document 4. Since this is bulky, a reaction intermediate represented by the following formula (6) becomes unstable, whereby the amount of an impurity represented by the formula (7) is apt to increase. The compound of the formula (7) was confirmed by HPLC and LC (liquid chromatography)/MS (mass spectrum).

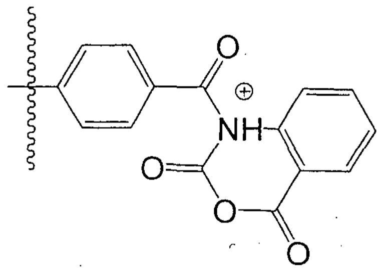

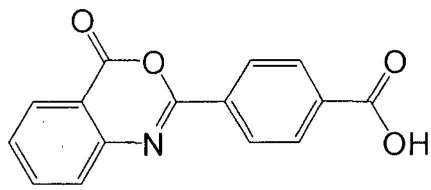

(6) (7)

The organic solvent is a solvent in which the anthranilic acid derivative (1) and the aromatic dicarboxylic acid dihalide (2) as the raw materials are soluble and which does not participate in the reaction. Specific examples of the organic solvent include ketones such as acetone, methyl isobutyl ketone (MIBK) and cyclohexanone, aromatic hydrocarbons such as toluene and xylene, ethers such as tetrahydrofuran and dioxane, and halogenated hydrocarbons such as 1,2-dichloroethane, 1,1,1-trichloroethane and chlorobenzene. Out of these, ketones are particularly preferred from the viewpoints of the solubility of the raw materials and reactivity.

The amount of the organic solvent used in the reaction is not particularly limited but preferably 350 to 3, 500 parts by weight, more preferably 800 to 1,200 parts by weight based on 100 parts by weight of the anthranilic acid derivative.

The present invention is characterized in that an inert gas is introduced into the reaction system to promote the reaction in an inert gas stream so as to remove hydrogen halide.

Examples of the inert gas used in the present invention include nitrogen and argon, out of which nitrogen is preferred. The amount of the inert gas used in the reaction is 1 to 50 liters/hr based on 1 mole of the anthralinic acid derivative. When the amount exceeds the above range, the amount dissipated by the inert gas stream becomes huge, and when the amount falls below the above range, the reaction proceeds very slowly. The amount of the inert gas is more preferably 5 to 15 liters/hr. The inert gas may be blown into the organic solvent or the gas phase of the reactor.

The reactor has an inert gas introduction unit, a gas lead-out unit and a reflux device and is capable of stirring an ordinary reaction solution in the form of slurry. In addition, a unit for removing hydrogen halide contained in the inert gas which has been let pass through the reactor is required. The reaction temperature is preferably room temperature to 140°C. When the reaction temperature is higher than 140°C, coloring tends to occur. It is more preferably 50 to 90°C. The reaction time which changes according to the flow rate of the inert gas and the reaction temperature is preferably 0.5 to 20 hours, more preferably 8 to 12 hours. The amide compound represented by the formula (3) can be used in the subsequent step without being purified.

In the step 1, the amide compound represented by the following formula (3) is obtained as a crystal.

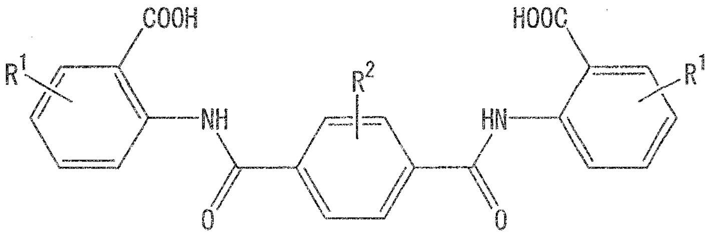

(3)

In the above formula, $R^1$ and $R^2$ are as defined in the above formulas (1) to (3).

(step 2)

The step 2 is to obtain a bisbenzoxazinone compound represented by the following formula (4) by reacting the amide compound represented by the formula (3) obtained in the step (1) with a dehydrating agent to cyclize it.

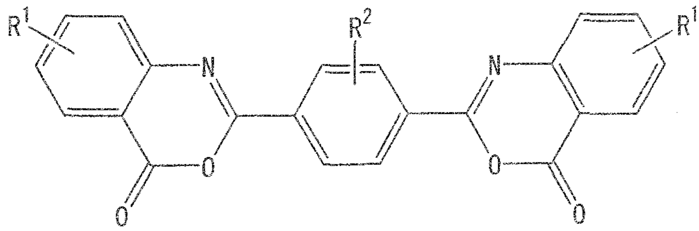

(4)

In the above formula, $R^1$ and $R^2$ are as defined in the above formulas (1) to (3). More preferably, $R^1$ and $R^2$ are each a hydrogen atom. A specific example of the compound is 2,2'-phenylenebis(3,1-benzoxazin-4-one).

Examples of the dehydrating agent include acetic anhydride, phosphorus pentoxide and sulfur trioxide, out of which acetic anhydride is preferred from the viewpoint of handling ease. At least 2 equivalents, preferably at least 5 equivalents, more preferably at least 20 equivalents of the dehydrating agent is required based on 1 equivalent of the amide compound represented by the formula (3). Preferably, the dehydrating agent may also be used as a solvent.

Any reactor is acceptable if it has a reflux device to stir the reaction solution in the form of slurry. Since part of an acidic gas formed during the reaction comes out from an exhaust port, a device for removing this is required. The reaction temperature differs according to the boiling point of the solvent in use but is preferably 80°C or higher, more preferably 120 to 140°C. Rinsing is preferably carried out to remove an acidic component by-produced during the dehydration reaction. The reaction time differs according to the equivalent and type of the dehydrating agent but is preferably 1 to 24 hours, more preferably 8 to 16 hours.

In the production method of the present invention, in the formulas (1) to (4), preferably, is a hydrogen atom, $R^2$ is a hydrogen atom, and X is a chlorine atom.

Since the reaction is prompted by the inert gas without using an alkali metal in the production method of the present invention, a bisbenzoxazinone compound having a low content of an alkali metal can be obtained. Further, since the reaction proceeds in an inert gas atmosphere, a side reaction such as oxidation hardly occurs during the reaction and a bisbenzoxazinone compound having high purity and a good color is obtained.

<resin composition>

The resin composition comprises 100 parts by weight of an aromatic polycarbonate (component A) and 0.01 to 5 parts by weight of the bisbenzoxazinone compound (component B) produced according to the present invention. The content of the bisbenzoxazinone compound (component B) is preferably 0.01 to 3 parts by weight, more preferably 0.1 to 1 part by weight based on 100 parts by weight of the aromatic polycarbonate (component A).

(aromatic polycarbonate: component A)

The aromatic polycarbonate used as the component A is obtained by reacting a diphenol with a carbonate precursor. Examples of the reaction include interfacial polymerization, melt transesterification, the solid-phase transesterification of a carbonate prepolymer and the ring-opening polymerization of a cyclic carbonate compound.

Typical examples of the diphenol used herein include hydroquinone, resorcinol, 4,4'-biphenol, 1,1-bis(4-hydroxyphenyl)ethane, 2,2-bis(4-hydroxyphenyl)propane (commonly known as "bisphenol A"), 2,2-bis(4-hydroxy-3-methylphenyl)propane, 2,2-bis(4-hydroxyphenyl)butane, 1,1-bis(4-hydroxyphenyl)-1-phenylethane, 1,1-bis(4-hydroxyphenyl)cyclohexane, 1,1-bis(4-hydroxyphenyl)-3,3,5-trimethylcyclohexane, 2,2-bis(4-hydroxyphenyl)pentane, 4,4'-(p-phenylenediisopropylidene)diphenol, 4,4'-(m-phenylenediisopropylidene)diphenol, 1,1-bis(4-hydroxyphenyl)-4-isopropylcyclohexane, bis(4-hydroxyphenyl)oxide, bis(4-hydroxyphenyl)sulfide, bis(4-hydroxyphenyl)sulfoxide, bis(4-hydroxyphenyl)sulfone, bis(4-hydroxyphenyl)ketone, bis(4-hydroxyphenyl)ester, 2,2-bis(3,5-dibromo-4-hydroxyphenyl)propane, bis(3,5-dibromo-4-hydroxyphenyl)sulfone, bis(4-hydroxy-3-methylphenyl)sulfide, 9,9-bis(4-hydroxyphenyl)fluorene and 9,9-bis(4-hydroxy-3-methylphenyl)fluorene. Out of these, bis(4-hydroxyphenyl)alkanes are preferred, and bisphenol A is particularly preferred from the viewpoint of impact resistance.

The carbonate precursor is a carbonyl halide, diester carbonate or haloformate, as exemplified by phosgene, diphenyl carbonate and dihaloformates of a diphenol.

For the manufacture of an aromatic polycarbonate resin from the above diphenol and the above carbonate precursor by interfacial polymerization, a catalyst, a terminal capping agent and an antioxidant for preventing the oxidation of the diphenol may be optionally used. The aromatic polycarbonate may be a branched polycarbonate obtained by copolymerizing a polyfunctional aromatic compound having 3 or more aromatic groups, a polyester carbonate resin obtained by copolymerizing an aromatic or aliphatic bifunctional carboxylic acid, or a mixture of two or more of the obtained polycarbonate resins.

Examples of the polyfunctional aromatic compound having 3 or more aromatic groups include 1,1,1-tris(4-hydroxyphenyl)ethane and 1,1,1-tris(3,5-dimethyl-4-hydroxyphenyl)ethane.

When the polyfunctional compound forming a branched polycarbonate is contained, its amount is preferably 0.001 to 1 mol%, more preferably 0.005 to 0.5 mol%, much more preferably 0.01 to 0.3 mol% based on the whole amount of the aromatic polycarbonate. Particularly in the case of melt transesterification, a branched structure may be produced as a side reaction. The amount of this branched structure is also preferably 0.001 to 1 mol%, more preferably 0.005 to 0.5 mol%, much more preferably 0.01 to 0.3 mol% based on the whole amount of the aromatic polycarbonate. The amount of the branched structure can be calculated by $^1$H-NMR measurement.

The aliphatic bifunctional carboxylic acid is preferably $\alpha,\omega$-dicarboxylic acid. Preferred examples of the aliphatic bifunctional carboxylic acid include linear saturated aliphatic dicarboxylic acids such as sebacic acid (decanedioic acid), dodecanedioic acid, tetradecanedioic acid, octadecanedioic acid and icosanedioic acid.

Further, a polycarbonate-polyorganosiloxane copolymer obtained by copolymerizing a polyorganosiloxane unit may also be used.

The reaction by the interfacial polymerization process is generally a reaction between a diphenol and phosgene in the presence of an acid binder and an organic solvent. Examples of the acid binder include alkali metal hydroxides such as sodium hydroxide and potassium hydroxide, and pyridine.

Examples of the organic solvent include halogenated hydrocarbons such as methylene chloride and chlorobenzene.

A catalyst such as a tertiary amine or a quaternary ammonium salt may be used to promote the reaction. A monofunctional phenol such as phenol, p-tert--butylphenol or p-cumylphenol is preferably used as a molecular weight control agent. Further examples of the monofunctional phenol include decyl phenol, dodecyl phenol, tetradecyl phenol, hexadecyl phenol, octadecyl phenol, eicosyl phenol, docosyl phenol and triacontyl phenol. These monofunctional phenols having a relatively long-chain alkyl group are effective when the improvement of fluidity and resistance to hydrolysis is desired.

The reaction temperature is generally 0 to 40°C, the reaction time is several minutes to 5 hours, and pH during the reaction is preferably kept at 10 or more.

The reaction by the melt process is generally a transesterification reaction between a diphenol and a diester carbonate. The diphenyl and the diester carbonate are mixed together in the presence of an inert gas and reacted with each other at 120 to 350°C under reduced pressure. The degree of depressurization is changed stepwise, and the final pressure is set to 133 Pa or less to remove the formed phenol to the outside of the system. The reaction time is generally about 1 to 4 hours.

Examples of the above diester carbonate include diphenyl carbonate, dinaphthyl carbonate, bis(diphenyl)carbonate, dimethyl carbonate, diethyl carbonate and dibutyl carbonate. Out of these, diphenyl carbonate is preferred.

A polymerization catalyst may be used to accelerate the polymerization rate. The polymerization catalyst is a catalyst which is generally used in an esterification reaction or a transesterification reaction, as exemplified by hydroxides of alkali metals and alkali earth metals such as sodium hydroxide and potassium hydroxide, boron and aluminum hydroxides, alkali metal salts, alkali earth metal salts, quaternary ammonium salts, alkoxides of alkali metals and alkali earth metals, organic acid salts of alkali metals and alkali earth metals, zing compounds, boron compounds, silicon compounds, germanium compounds, organic tin compounds, lead compounds, antimony compounds, manganese compounds, titanium compounds and zirconium compounds. These catalysts may be used alone or in combination of two

or more. The amount of the polymerization catalyst is preferably $1 \times 10^{-8}$ to $1 \times 10^{-3}$ equivalent, more preferably $1 \times 10^{-7}$ to $5 \times 10^{-4}$ equivalent based on 1 mole of the diphenol as one of the raw materials.

In the polymerization reaction, to reduce the number of phenolic terminal groups, a compound such as 2-chlorophenylphenyl carbonate, 2-methoxycarbonylphenylphenyl carbonate or 2-ethoxycarbonylphenylphenyl carbonate may be added in the latter stage or at the end of the polycondensation reaction.

Further, a deactivator is preferably used to neutralize the activity of the catalyst in the melt transesterification process. The amount of the deactivator is preferably 0.5 to 50 moles based on 1 mole of the residual catalyst. The deactivator is used in an amount of preferably 0.01 to 500 ppm, more preferably 0.01 to 300 ppm, much more preferably 0.01 to 100 ppm based on the aromatic polycarbonate after polymerization. Preferred examples of the deactivator include phosphonium salts such as tetrabutylphosphonium dodecylbenzenesulfonate and ammonium salts such as tetraethylammonium dodecylbenzylsulfate.

The viscosity average molecular weight of the aromatic polycarbonate is preferably 13,000 to 40,000, more preferably 14,000 to 30,000, much more preferably 15,000 to 27,000. When an aromatic polycarbonate having the above viscosity average molecular weight is used, the resin composition has sufficiently high strength and high melt fluidity at the time of molding. The high melt fluidity is preferred because it enables the further reduction of molding strain. Within the above range, resistance to secondary processing such as hard coating becomes satisfactory. The aromatic polycarbonate may be obtained by mixing a polycarbonate having a viscosity average molecular weight outside the above range.

The viscosity average molecular weight (M) of the aromatic polycarbonate is obtained by inserting the specific viscosity ($\eta_{sp}$) of a solution prepared by dissolving 0.7 g of the polycarbonate resin in 100 ml of methylene chloride at 20°C into the following equation.

$$\eta_{sp}/c = [\eta] + 0.45 \times [\eta]^2 c \quad ([\eta] \text{ represents an intrinsic viscosity})$$

$$[\eta] = 1.23 \times 10^{-4} M^{0.83}$$

$$c = 0.7$$

(stabilizer: component C)

The resin composition may contain a stabilizer (component C). Preferably, the stabilizer (component C) is at least one selected from a phosphorus-based stabilizer (component C1) and a hindered phenol-based stabilizer (component C2).

The phosphorus-based stabilizer (component C1) is selected from a phosphite compound, a phosphate compound, a phosphonite compound and a phosphonate compound.

Specific examples of the phosphite compound include triphenyl phosphite, tris(nonylphenyl)phosphite, tridecyl phosphite, trioctyl phosphite, trioctadecyl phosphite, didecylmonophenyl phosphite, dioctylmonophenyl phosphite, diisopropylmonophenyl phosphite, monobutyldiphenyl phosphite, monodecyldiphenyl phosphite, monooctyldiphenyl phosphite, 2,2-methylenebis(4,6-di-tert-butylphenyl)octyl phosphite, tris(diethylphenyl)phosphite, tris(di-iso-propylphenyl)phosphite, tris(di-n-butylp-henyl)phosphite, tris(2,4-di-tert-butylphenyl)phosphite, tris(2,6-di-tert-butylphenyl)phosphite, distearyl pentaerythritol diphosphite, bis(2,4-di-tert-butylphenyl)pentaerythritol diphosphite, bis(2,6-di-tert-butyl-4-methylphenyl)pentaerythritol diphosphite, bis(2,6-di-tert-butyl-4-ethylphenyl)pentaerythritol diphosphite, phenyl bisphenol A pentaerythritol diphosphite, bis(nonylphenyl)pentaerythritol diphosphite and dicyclohexylpentaerythritol diphosphite.

Other phosphite compounds having a cyclic structure which reacts with a diphenol may also be used. The other phosphite compounds include
2,2'-methylenebis(4,6-di-tert-butylphenyl)(2,4-di-tert-b utylphenyl)phosphite,
2,2'-methylenebis(4,6-di-tert-butylphenyl)(2-tert-butyl-4-methylphenyl)phosphite,
2,2'-methylenebis(4-methyl-6-tert-butylphenyl)(2-tert-bu tyl-4-methylphenyl)phosphite and
2,2'-ethylidenebis(4-methyl-6-tert-butylphenyl)(2-tert-b utyl-4-methylphenyl)phosphite.

Examples of the phosphate compound include tributyl phosphate, trimethyl phosphate, tricresyl phosphate, triphenyl phosphate, trichlorophenyl phosphate, triethyl phosphate, diphenylcresyl phosphate, diphenylmonoorthoxenyl phosphate, tributoxyethyl phosphate, dibutyl phosphate, dioctyl phosphate and diisopropyl phosphate. Out of these,

triphenyl phosphate and trimethyl phosphate are preferred.

Examples of the phosphonite compound include tetrakis(2,4-di-tert-butylphenyl)-4,4'-biphenylene diphosphonite, tetrakis(2,4-di-tert-butylphenyl)-4,3'-biphenylene diphosphonite, tetrakis(2,4-di-tert-butylphenyl)-3,3'-biphenylene diphosphonite, tetrakis(2,6-di-tert-butylphenyl)-4,4'-biphenylene diphosphonite, tetrakis(2,6-di-tert-butylphenyl)-4,3'-biphenylene diphosphonite, tetrakis(2,6-di-tert-butylphenyl)-3,3'-biphenylene diphosphonite, bis(2,4-di-tert-butylphenyl)-4-phenyl-phenyl phosphonite bis(2,4-di-tert-butylphenyl)-3-phenyl-phenyl phosphonite bis(2,6-di-n-butylphenyl)-3-phenyl-phenyl phosphonite, bis(2,6-di-tert-butylphenyl)-4-phenyl-phenyl phosphonite and bis(2,6-di-tert-butylphenyl)-3-phenyl-phenyl phosphonite. Tetrakis(di-tert-butylphenyl)-biphenylene diphosphonites and bis(di-tert-butylphenyl)-phenyl-phenyl phosphonites are preferred, and tetrakis(2,4-di-tert-butylphenyl)-biphenylene diphosphonite and bis(2,4-di-tert-butylphenyl)-phenyl-phenyl phosphonite are more preferred. The phosphonite compound may be and is preferably used in combination with the above phosphite compound having an aryl group with two or more alkyl substituents.

Examples of the phosphonate compound include dimethyl benzenephosphonate, diethyl benzenephosphonate and dipropyl benzenephosphonate.

These phosphorus-based stabilizers (component C1) may be used alone or in combination of two or more. The phosphorus-based stabilizer (component C1) is preferably a phosphite compound or a phosphonite compound. Tris(2,4-di-tert-butylphenyl)phosphite, tetrakis(2,4-di-tert-butylphenyl)-4,4'-biphenylene diphosphonite and bis(2,4-di-tert-butylphenyl)-phenyl-phenyl phosphonite are particularly preferred. A combination of one of them and a phosphate compound is also preferred.

The content of the phosphorus-based stabilizer (component C1) is preferably 0.0001 to 1 part by weight, more preferably 0.001 to 0.1 part by weight, much more preferably 0.005 to 0.1 part by weight based on 100 parts by weight of the aromatic polycarbonate (component A). When the content of the phosphorus-based stabilizer (component C1) falls below the above range, it is difficult to obtain a satisfactory stabilizing effect and when the content exceeds the above range, the physical properties of the composition may deteriorate.

Examples of the hindered phenol-based stabilizer (component C2) include α-tocopherol, butylhydroxytoluene, sinapyl alcohol, vitamin E, n-octadecyl-β-(4'-hydroxy-3',5'-di-tert-butylphenyl)prop ionate, 2-tert-butyl-6-(3'-tert-butyl-5'-methyl-2'-hydroxybenzyl )-4-methylphenyl acrylate, 2,6-di-tert-butyl-4-(N,N-dimethylaminomethyl)phenol, 3,5-di-tert-butyl-4-hydroxybenzylphosphonate diethyl ester, 2,2'-methylenebis(4-methyl-6-tert-butylphenol), 2,2'-methylenebis(4-ethyl-6-tert-butylphenol), 4,4'-methylenebis(2,6-di-tert-butylphenol), 2,2'-methylenebis(4-methyl-6-cyclohexylphenol), 2,2'-dimethylene-bis(6-α-methyl-benzyl-p-cresol), 2,2'-ethylidene-bis(4,6-di-tert-butylphenol), 2,2'-butylidene-bis(4-methyl-6-tert-butylphenol), 4,4'-butylidene-bis(3-methyl-6-tert-butylphenol), triethylene glycol-N-bis-3-(3-tert-butyl-4-hydroxy-5-methylphenyl) propionate, 1,6-hexanediol bis[3-(3,5-di-tert-butyl-4-hydroxyphenyl)propionate], bis[2-tert-butyl-4-methyl 6-(3-tert-butyl-5-methyl-2-hydroxybenzyl)phenyl] terephthalate, 3,9-bis{2-[3-(3-tert-butyl-4-hydroxy-5-methylphenyl) propionyloxy]-1,1-dimethylethyl}-2,4,8,10-tetraoxaspiro [5,5]undecane, 4,4'-thiobis(6-tert-butyl-m-cresol), 4,4'-thiobis(3-methyl-6-tert-butylphenol), 2,2'-thiobis(4-methyl-6-tert-butylphenol), bis(3,5-di-tert-butyl-4-hydroxybenzyl) sulfide, 4,4'-di-thiobis(2,6-di-tert-butylphenol), 4,4'-tri-thiobis(2,6-di-tert-butylphenol), 2,2-thiodiethylenebis-[3-(3,5-di-tert-butyl-4-hydroxyphenyl)propionate], 2,4-bis(n-octylthio)-6-(4-hydroxy-3',5'-di-tert-butylanilino)-1,3,5-triazine, N,N'-hexamethylenebis-(3,5-di-tert-butyl-4-hydroxyhydrocinnamide), N,N'-bis[3-(3,5-di-tert-butyl-4-hydroxyphenyl)propionyl] hydrazine, 1,1,3-tris(2-methyl-4-hydroxy-5-tert-butylphenyl)butane, 1,3,5-trimethyl-2,4,6-tris(3,5-di-tert-butyl-4-hydroxybenzyl)benzene, tris(3,5-di-tert-butyl-4-hydroxyphenyl)isocyanurate, tris(3,5-di-tert-butyl-4-hydroxybenzyl)isocyanurate, 1,3,5-tris(4-tert-butyl-3-hydroxy-2,6-dimethylbenzyl) isocyanurate, 1,3,5-tris 2[3(3,5-di-tert-butyl-4-hydroxyphenyl)propionyloxy]ethyl isocyanurate and tetrakis[methylene-3-(3',5'-di-tert-butyl-4-hydroxyphenyl)propionate]methane. All of them are easily acquired. The above hindered phenol-based antioxidants may be used alone or in combination of two or more.

The content of the hindered phenol-based stabilizer (component (2) is preferably 0.0001 to 1 part by weight, more preferably 0.0 01 to 0.1 part by weight, much more preferably 0.005 to 0.1 part by weight based on 100 parts by weight of the aromatic polycarbonate (component A). When the content of the hindered phenol-based stabilizer (component C2) falls below the above range, it is difficult to obtain a satisfactory stabilizing effect and when the content exceeds the above range, the composition is colored, thereby deteriorating the appearance of a molded product thereof.

(optical stabilizer)

The resin composition may also contain a hindered amine-based optical stabilizer. Examples of the hindered amine-based optical stabilizer include bis(2,2,6,6-tetramethyl-4-piperidyl)sebacate, bis(1,2,2,6,6-pentamethyl-4-piperidyl)sebacate, tetrakis(2,2,6,6-tetramethyl-4-piperidyl)-1,2,3,4-butane tetracarboxylate, tetrakis(1,2,2,6,6-pentamethyl-4-piperidyl)-1,2,3,4-butane tetracarboxylate, poly{[6-(1,1,3,3-tetramethylbutyl)amino-1,3,5-triazine-2,4-diyl][(2,2,6,6-tetramethylpiperidyl)imino]hexamethyl ene[(2,2,6,6-tetramethylpiperidyl)imino]} and polymethylpropyl 3-oxy-[4-(2,2,6,6-tetramethyl) piperidinyl]siloxane.

The above optical stabilizers may be used alone or in combination of two or more. The content of the optical stabilizer is preferably 0.0005 to 3 parts by weight, more preferably 0.01 to 2 parts by weight, much more preferably 0.02 to 1 part by weight based on 100 parts by weight of the aromatic polycarbonate (component A).

<anthraquinone-based dye: component D>

The resin composition may contain an anthraquinone-based dye having no OH functional group in the skeleton. Dyes which are generally used as bluing agents by people having ordinary skill in the art may be used as the anthraquinone-based dye having no OH

functional group in the skeleton, and not only blue dyes but also red, orange, green, yellow and purple dyes may be used. A wide variety of colors can be obtained by using these dyes alone or in combination.

Examples of the anthraquinone-based dye having no OH functional group in the skeleton (component D) include the MACROLEX Violet B (compound of the formula (a)) and MACROLEX Blue RR (compound of the formula (b)) of Bayer AG, the PLAST Blue 8520 (compound of the formula (c)), PLAST Violet 8855 (compound of the formula (d)), PLAST Red 8350, PLAST Red 8340, PLAST Red 8320 and OIL Green 5602 of Arimoto Chemical Co., Ltd., the DIARESIN Blue N of Mitsubishi Chemical Corporation and the SUMIPLAST Violet RR of Sumitomo Chemical Co., Ltd. The compounds of the formulas (a), (b), (c) and (d) are preferably used.

The content of the anthraquinone-based dye (component D) is preferably 1 x $10^{-6}$ so 1,000 x $10^{-6}$ part by weight, more preferably 5 x $10^{-6}$ to 500 x $10^{-6}$ part by weight, much more preferably 10 x $10^{-6}$ to 150 x $10^{-6}$ part by weight, particularly preferably 10 x $10^{-6}$ to 100 x $10^{-6}$ part by weight based on 100 parts by weight of the aromatic polycarbonate (component A).

A dye except for anthraquinone-based dyes may be used in combination but the anthraquinone-based dye having no OH functional group in the skeleton desirably accounts for 50 % or more of the total of all the dyes. When this anthraquinone-based dye is used in combination with another dye, the total content of the dyes is 1 x $10^{-6}$ to 1,000 x $10^{-6}$ part by weight based on 100 parts by weight of the aromatic polycarbonate (component A).

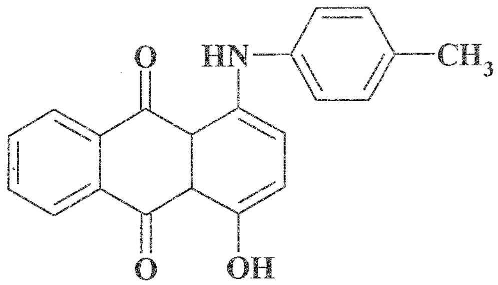

(a) (MACROLEX Violet B)

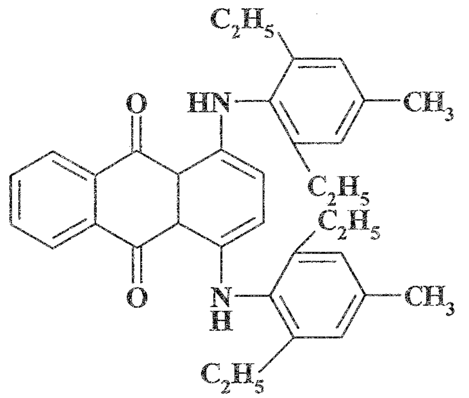

(b) (MACROLEX Blue RR)

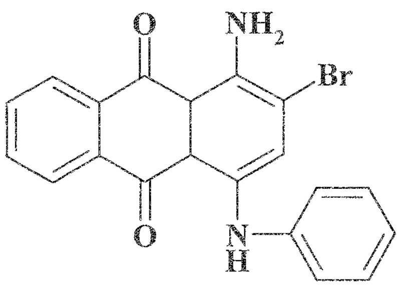

(c) (PLAST Blue 8520)

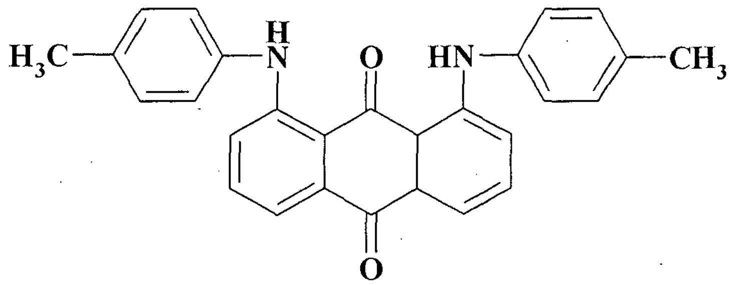

(d) (PLAST Violet 8855)

(dye and pigment)

The resin composition may contain a dye and a pigment except for the anthraquinone-based dye (component D) as long as the object of the present invention is not impaired. A dye is preferred because it keeps transparency. Preferred examples of the dye include perylene-based dyes, coumarin-based dyes, thioindigo-based dyes, anthraquinone-based dyes, thioxanthone-based dyes, ferrocyanides such as iron blue pigment, perinone-based dyes, quinoline-based dyes, quinacridone-based dyes, dioxazine-based dyes, isoindolinone-based dyes and phthalocyanine-based dyes. The total content of the dye and the pigment is preferably 0.0001 to 1 part by weight, more preferably 0.0005 to 0.5 part by weight based on 100 parts by weight of the aromatic polycarbonate (component A).

(release agent)

The resin composition may contain a release agent. The release agent is preferably an ester (fatty acid ester) of an alcohol and an aliphatic carboxylic acid.

The alcohol may be either a monohydric alcohol or a polyhydric alcohol having 2 or more hydroxyl groups. The polyhydric alcohol is preferred as the alcohol. Examples of the monohydric alcohol include dodecanol, tetradecanol, hexadecanol, octadecanol, eicosanol, tetracosanol, ceryl alcohol and triacontanol. Examples of the polyhydric alcohol include glycerin, diglycerin, polyglycerin (such as decaglycerin), pentaerythritol, dipentaerythritol, diethylene glycol and

propylene glycol. Out of these, pentaerythritol is preferred.

The aliphatic carboxylic acid has preferably 3 to 32 carbon atoms, particularly preferably 10 to 22 carbon atoms. Examples of the aliphatic carboxylic acid include saturated aliphatic carboxylic acids such as decanoic acid, undecanoic acid, dodecanoic acid, tridecanoic acid, tetradecanoic acid, pentadecanoic acid, hexadecanoic acid (palmitic acid), heptadecanoic acid, octadecanoic acid (stearic acid), nonadecanoic acid, icosanoic acid and docosanoic acid (behenic acid). Unsaturated aliphatic carboxylic acids such as palmitoleic acid, oleic acid, linoleic acid, linoleic acid, eicosanoic acid, eicosapentaenoic acid and cetoleic acid are also included. Out of these, aliphatic carboxylic acids having 14 to 20 carbon atoms are preferred. Saturated aliphatic carboxylic acids are particularly preferred.

Since these aliphatic carboxylic acids are generally produced from natural oils and fats such as animal oils and fats (beef tallow and lard) and vegetable oils and fats (such as palm oil), they are mixtures containing another carboxylic acid component having a different number of carbon atoms. Therefore, the aliphatic carboxylic acid is in the form of a mixture containing another carboxylic acid component produced from a natural oil or fat.

The acid value of the fatty acid ester is preferably 20 or less (can be substantially "0'"). However, a full ester preferably contains a free fatty acid in no small measure to improve releasability. In this respect, the acid value of the full ester is preferably 3 to 15. The iodine value of the fatty acid ester is preferably 10 or less (can be substantially "0"). These properties can be obtained by methods specified in JIS K0070.

The fatty acid ester may be a partial ester or a full ester but preferably a partial ester in order to obtain high releasability and durability. A glycerin monoester is particularly preferred. The glycerin monoester comprises a monoester of glycerin and a fatty acid as the main component. Preferred examples of the fatty acid include saturated fatty acids such as stearic acid, palmitic acid, behenic acid, arachidic acid, montanic acid and lauric acid. Unsaturated fatty acids such as oleic acid, linoleic acid and sorbic acid are also included. A fatty acid ester comprising a glycerin monoester of stearic acid, behenic acid or palmitic acid as the main component is particularly preferred. The fatty acid is synthesized from a natural fatty acid and becomes a mixture as described above. The glycerin monoester may be used in combination with another release agent, especially a fatty acid full ester. Even when the glycerin monoester is used in combination with another release agent, the glycerin monoester is preferably the main component of the release agent. That is, the content of the glycerin monoester is preferably not less than 60 wt%, more preferably not less than 80 wt%, much more preferably not less than 85 wt% based on 100 wt% of the release agent. Therefore, the release agent is preferably a full ester of a polyhydric alcohol and an aliphatic carboxylic acid. The esterification rate of the full ester in the present invention does not always need to be 100 % and may be not less than 80 %, preferably not less than 85 %.

(antistatic agent)

The resin composition may contain an antistatic agent. An example of the antistatic agent is (i) an organic phosphonium sulfonate such as a phosphonium aryl sulfonate typified by phosphonium dodecylbenzenesulfonate, or a phosphonium alkyl sulfonate. The content of the organic phosphonium sulfonate is preferably not more than 5 parts by weight, more preferably 0.05 to 5 parts by weight, much more preferably 1 to 3.5 parts by weight, particularly preferably 1.5 to 3 parts by weight based on 100 parts by weight of the aromatic polycarbonate (component A).

Another example of the antistatic agent is (ii) an organic alkali (earth) metal sulfonate such as organic lithium sulfonate, organic sodium sulfonate, organic potassium sulfonate, organic cesium sulfonate, organic rubidium sulfonate, organic calcium sulfonate, organic magnesium sulfonate or organic barium sulfonate. Specific examples of the organic alkali (earth) metal sulfonate include metal salts of dodecylbenzenesulfonic acid and metal salts of perfluoroalkanesulfonic acid. The content of the organic alkali (earth) metal sulfonate is preferably not more than 0.5 part by weight, more preferably 0.001 to 0.3 part by weight, much more preferably 0.005 to 0.2 part by weight based on 100 parts by weight of the aromatic polycarbonate (component A). Alkali metal salts such as potassium, cesium and rubidium are preferred.

Still another example of the antistatic agent is (iii) an organic ammonium sulfonate such as ammonium alkyl sulfonate or ammonium aryl sulfonate. The content of the ammonium salt is preferably not more than 0.05 part by weight based on 100 parts by weight of the aromatic polycarbonate (component A).

A further example of the antistatic agent is (iv) a glycerin derivative ester such as glycerin monostearate, maleic anhydride monoglyceride or maleic anhydride diglyceride. The content of the ester is preferably not more than 0.5 part by weight based on 100 parts by weight of the aromatic polycarbonate (component A).

A still further example of the antistatic agent is (v) a polymer containing a poly(oxyalkylene)glycol component such as polyether ester amide. The content of the polymer is preferably not more than 5 parts by weight based on 100 parts by weight of the aromatic polycarbonate (component A).

Other examples of the antistatic agent include (vi) non-organic compounds such as carbon black, carbon fiber, carbon nanotube, graphite, metal powders and metal oxide powders. The content of the non-organic compound is preferably not more than 0.05 part by weight based on 100 parts by weight of the aromatic polycarbonate (component A).

(flame retardant)

The resin composition may contain a flame retardant as long as the object of the present invention is not impaired. The flame retardant is selected from a halogenated bisphenol A polycarbonate-based flame retardant, an organic salt-based flame retardant, an aromatic phosphate-based flame retardant and a halogenated aromatic phosphate-based flame retardant. At least one of these flame retardants can be used.

Examples of the halogenated bisphenol A polycarbonate-based flame retardant include tetrabromobisphenol A polycarbonate-based flame retardants and tetrabromobisphenol A-bisphenol A copolycarbonate-based flame retardants.

Examples of the organic salt-based flame retardant include dipotassium diphenylsulfone-3,3'-disulfonate, potassium diphenylsulfone-3-sulfonate, sodium 2,4,5-trichlorobenzenesulfonate, potassium 2,4,5-trichlorobenzenesulfonate, potassium bis(2,6-dibromo-4-cumylphenyl)phosphate, sodium bis(4-cumylphenyl)phosphate, potassium bis(p-toluenesulfone)imide, potassium bis(diphenylphosphate)imide, potassium bis(2,4,6-tribromophenyl)phosphate, potassium bis(2,4-dibromophenyl)phosphate, potassium bis(4-bromophenyl)phosphate, potassium diphenylphosphate, sodium diphenylphosphate, potassium perfluorobutanesulfonate, sodium or potassium laurylsulfate and sodium or potassium hexadecylsulfate.

Examples of the halogenated aromatic phosphate-based flame retardant include tris(2,4,6-tribromophenyl)phosphate, tris(2,4-dibromophenyl)phosphate and tris(4-bromophenyl)phosphate.

Examples of the aromatic phosphate-based flame retardant include triphenyl phosphate, tris(2,6-xylyl)phosphate, tetrakis(2,6-xylyl)resorcin diphosphate, tetrakis(2,6-xylyl)hydroquinone diphosphate, tetrakis(2,6-xylyl)-4,4'-biphenol diphosphate, tetraphenylresorcin diphosphate, tetraphenylhydroquinone diphosphate, tetraphenyl-4,4'-biphenol diphosphate, aromatic polyphosphates having no phenolic OH group, obtained from resorcin and phenol as aromatic ring sources, aromatic polyphosphates having a phenolic OH group, obtained from resorcin and phenol as aromatic ring sources, aromatic polyphosphates having no phenolic OH group, obtained from hydroquinone and phenol as aromatic ring sources, aromatic polyphosphates having a phenolic OH group, obtained from hydroquinone and phenol as aromatic ring sources ("aromatic polyphosphates" hereinbelow means both aromatic polyphosphates having a phenolic OH group and aromatic polyphosphates having no phenolic OH group), aromatic polyphosphates obtained from bisphenol A and phenol as aromatic ring sources, aromatic polyphosphates obtained from tetrabromobisphenol A and phenol as aromatic ring sources, aromatic polyphosphates obtained from resorcin and 2,6-xylenol as aromatic ring sources, aromatic polyphosphates obtained from hydroquinone and 2,6-xylenol as aromatic ring sources, aromatic polyphosphates obtained from bisphenol A and 2,6-xylenol as aromatic ring sources, and aromatic polyphosphates obtained from tetrabromobisphenol A and 2,6-xylenol as aromatic ring sources.

(others)

The resin composition may contain small amounts of another resin and an elastomer as long as the object of the present invention is not impaired.

Examples of the another resin include polyester resins such as polyethylene terephthalate and polybutylene terephthalate, polyamide resins, polyimide resins, polyether imide resins, polyurethane resins, silicone resins, polyphenylene ether resins, polyphenylene sulfide resins, polysulfone resins, polyolefin resins such as polyethylene and polypropylene, polystyrene resins, acrylonitrile/styrene copolymer (AS resin), acrylonitrile/butadiene/styrene copolymer (ABS resin), polymethacrylate resins, phenolic resins and epoxy resins.

Examples of the elastomer include isobutylene/isoprene rubber, styrene/butadiene rubber, ethylene/propylene rubber, acrylic elastomers, polyester-based elastomers, polyamide-based elastomers, core-shell type elastomers such as MBS (methyl methacrylate/styrene/butadiene) rubber and MAS (methyl methacrylate/acrylonitrile/styrene) rubber.

The resin composition may contain an inorganic filler, a fluidity modifier, an antibacterial agent, an optical catalyst-based anti-fouling agent, an infrared absorbent and a photochromic agent.

(characteristic properties of resin composition)

The resin composition has a total light transmittance of 70 % or more when it is molded into a 2 mm-thick plate.

The resin composition has a $\Delta$haze before and after a moist heat test of preferably 2.0 or less, more preferably 1.5 or less, much more preferably 1.0 or less when it is molded into a 2 mm-thick plate.

The resin composition has a $\Delta$haze before and after a weatherability promotion test of preferably 1.0 or less, more preferably 0.8 or less, much more preferably 0. 6 or less when it is molded into a 2 mm-thick plate.

The maximum peak of the light reflectance at 380 to 480 nm of the resin composition measured by placing a white standard plate on a 2 mm-thick plate molded from the resin composition is preferably 60 to 80 %, more preferably 65 to 75 %, much more preferably 65 to 72 %.

(production of resin composition)

To produce the resin composition, any method is employed. For example, the aromatic polycarbonate (component A) and the bisbenzoxazinone compound (component B) and optionally other additives are fully mixed together by means of pre-mixing means such as a twin-cylinder mixer, Henschel mixer, mechanochemical device or extrusion mixer, optionally granulated by means of an extrusion granulator or briquetting machine, melt kneaded by means of a melt kneader typified by a vented double-screw extruder and then pelletized by means of a device such as a pelletizer.
Alternatively, the above components are supplied into a melt kneader typified by a vented double-screw extruder independently, or after some of the above components are pre-mixed together, the obtained pre-mixture is supplied into the melt kneader independently of the other components. To premix some of the above components, after components except for the aromatic polycarbonate (component A) are pre-mixed together, the resulting pre-mixture is mixed with the aromatic polycarbonate (component A).
To pre-mix the above components, when the component A is powdery, part of the powdery component A is blended with the additives to prepare a master batch of the additives diluted with the powder. Further, one component may be supplied from a halfway position of the melt extruder independently. When the components to be mixed together include a liquid component, a liquid injector or a liquid adder may be used to supply it into the melt extruder.
An extruder having a vent from which water contained in the raw material and a volatile gas generated from the molten kneaded resin can be removed may be preferably used. A vacuum pump is preferably installed to discharge the generated water and volatile gas to the outside of the extruder from the vent efficiently. A screen for removing foreign matter contained in the extruded raw material may be installed in a zone before the dice of the extruder to remove the foreign matter from the resin composition. Examples of the screen include a metal net, a screen changer and a sintered metal plate (such as a disk filter).
Examples of the melt kneader include a Banbury mixer, a kneading roll, a single-screw extruder and a multi-screw extruder having 3 or more screws, besides the double-screw extruder.
The resin extruded as described above is pelletized by directly cutting it or by forming it into a strand and cutting the strand with a pelletizer. When the influence of extraneous dust must be reduced at the time of pelletization, the atmosphere surrounding the extruder is preferably made clean.

<molded article>

There can be provided a molded article of the resin composition. The molded article can be obtained by injection molding a pellet of the resin composition. For injection molding, not only ordinary molding techniques but also injection compression molding, injection press molding, gas assist injection molding, insert molding, in-mold coating molding, insulated runner molding, quick heating and cooling molding, two-color molding, sandwich molding and super high-speed injection molding techniques may be employed. The cold runner system and the hot runner system may be selected for molding.
The molded article includes an extrusion molded article such as a sheet or a film. For the molding of a sheet or a film, an inflation, calendering or casting process may be used. Further, the resin composition may be molded into a heat shrinkable tube by carrying out specific stretching operation. The resin composition can be formed into a hollow molded article by rotational molding or blow molding.
The molded article is suitable for use as a transparent member which requires high quality as it is excellent in transparency, color, releasability, dry heat resistance and weatherability.
Examples of the transparent member include car transparent members such as head lamp lenses, winker lamp lenses, tail lamp lenses, resin windowpanes and meter covers. Head lamp lenses, more specifically plain head lamp lenses are particularly preferred. The plain head lamp lenses include a cover for lamps which have a reflector with a light condensing function, a lamp unit cover having a lamp unit as an integral unit and the like. Illuminating lamp covers, construction resin windowpanes, solar cell covers, solar cell substrates, display lenses and touch panels are also enumerated as the transparent members. Front covers for play machines such as pachinko machines, circuit covers, chassis and pachinko ball carrying guides are also included.
Various surface treatments may be made on the molded article. The surface treatments include hard coating, water-repelling and oil-repelling coating, hydrophilic coating, antistatic coating, UV absorption coating, infrared absorption coating and metallizing (such as deposition).
Since the molded article is excellent in initial color, molding heat resistance, moist heat resistance and weatherability and has improved resistance to cracking which occurs when it is exposed to a bad environment after molding, it is very suitable for the above surface treatments. The resin composition is suitable for a surface treatment which includes a factor having a bad influence upon a polycarbonate resin, such as a solvent, particularly hard coating.
Various hard coating agents may be used for hard coating. The hard coating agents include silicone resin-based hard coating agents and organic resin-based hard coating agents.

The silicone resin-based hard coating agents are resins having a siloxane bond. The resins include a partial hydrolysate of a trialkoxysilane, a tetraalkoxysilane or an alkylated product thereof, a hydrolysate of a mixture of methyl trialkoxysilane and phenyl trialkoxysilane, and a partially hydrolyzed condensate of a colloidal silica charged organotrialkoxysilane. Although they contain an alcohol which is produced during a condensation reaction, they may be further dissolved or dispersed into any organic solvent, water or a mixture thereof as required. Examples of the organic solvent for this purpose include lower fatty acid alcohols, polyhydric alcohols, and ethers and esters thereof. A surfactant such as a siloxane-based or alkyl fluoride-based surfactant may be added to obtain a hard coating layer having a smooth surface.

The organic resin-based hard coating agents include melamine resins, urethane resins, alkyd resins, acrylic resins and polyfunctional acrylic resins. The polyfunctional acrylic resins include polyol acrylates, polyester acrylates, urethane acrylates, epoxy acrylates and phosphazene acrylates. Out of these hard coating agents, silicone resin-based hard coating agents having high long-term weatherability and relatively high surface hardness are preferred, and it is particularly preferred that a cured layer formed from a silicone resin-based hard coating agent should be formed on a primer layer formed from a resin.

Examples of the resin for forming the primer layer include urethane resins comprising a block isocyanate component and a polyol component, acrylic resins, polyester resins, epoxy resins, melamine resins, amino resins, and polyfunctional acrylic resins such as polyester acrylates, urethane acrylates, epoxy acrylates, phosphazene acrylates, melamine acrylates and amino acrylates. They may be used alone or in combination of two or more. Out of these, a hard coating agent comprising preferably not less than 50 wt%, more preferably not less than 60 wt% of acrylic resin or polyfunctional acrylic resin is preferred, and a hard coating agent consisting of acrylic resin is particularly preferred.

Further, additives such as the above-described optical stabilizer and UV absorbent, a catalyst for silicone resin-based hard coating agents, a thermal or optical polymerization initiator, a polymerization inhibitor, a silicone antifoamer, a leveling agent, a thickener, a precipitation inhibitor, a dripping inhibitor, a flame retardant and an organic or inorganic pigment or dye as well as addition aids may be contained in the resin for forming the primer layer for the silicone resin-based hard coating agents.

Examples

The following examples are provided to further illustrate the present invention. The examples were evaluated by the following methods.

<bisbenzoxazinone compound>

(1) HPLC (high-performance liquid chromatography) purity

(i) anthranilic acid and isatoic anhydride
The purities of the above substances were measured with the LC10 System of Shimadzu Corporation under the following conditions.
Column: Inertsil ODS-3 of GL Science Inc. (4.6 mm in diameter x 2 5 0 mm)
Column temperature: 40°C.
Eluant: 10 mM sodium phosphate buffer solution having a pH of 2.6/acetonitrile = 50/50 (V/V)
Flow rate: 0.5 ml/min
Detector: 265 nm
Amount of injected sample: 10 μl
(ii) 2,2'-phenylenebis(3,1-benzoxazin-4-one)
The purity of the above substance was measured with the LC10 System of Shimadzu Corporation under the following conditions.
Column: ZORBAX SB-C18 of Agilent Technologies Inc. (4.6 mm in diameter x 250 mm)
Column temperature: 40°C
Eluant A: 10 mM sodium phosphate buffer solution having a pH of 2.6/acetonitrile = 50/50 (V/V)
Eluant B: acetonitrile
Flow rate: 1 ml/min

Gradient condition:

| Time (min) | 0 | 15 | 40 | 50 | 60 | 80 |
|---|---|---|---|---|---|---|
| A(%) | 100 | 20 | 20 | 10 | 100 | 100 |

(continued)

| | | | | | | |
|---|---|---|---|---|---|---|
| B(%) | 0 | 80 | 80 | 90 | 0 | 0 |

Amount of injected sample: 5 μl
About 5 mg of the sample dissolving solvent was dissolved in about 15 ml of THF, the eluant A (acetonitrile/phosphate buffer solution) was added to the resulting solution to prepare 25 ml of the solution, and insoluble matter was filtered with a syringe filter to be analyzed.
The peak area of HPLC was converted into wt%.

(2) maximum light reflectance of powders

Powdery 2,2'-phenylenebis(3,1-benzoxazin-4-one) (compounds B1 to B3, comparative products 1 and 2) was charged into a cylindrical glass container having a diameter of 30 mm and a height of 13 mm to measure the reflectance of the powders with the TR-1800MK-II of Tokyo Denshoku Co., Ltd. As for the measurement conditions, the reflection method (2 degree field of view, illuminant C) was employed to measure the maximum wavelength at 380 to 780 nm.

(3) particle size distribution and average particle diameter of powders

The particle size distribution and average particle diameter of the powders were measured with the LA-920 laser diffraction/scattering particle size distribution measuring device of Horiba, Ltd. under the following conditions.
Measurement method: flow system
Dispersant: isopropyl alcohol
Supersonic wave: 10 minutes (intensity of 7)
Relative diffraction index: 116a000I
Circulation speed: 7

(4) content of metal sodium

The content of metal sodium was obtained by measuring a sample solution prepared by adding sulfuric acid to a sample, heating it to ash it and dissolving the residue in hydrochloric acid with the AA-220FS flame atomic absorptiometer of Varian Inc.

(5) YI value

The YI value was obtained from the following equation by charging powdery 2,2'-phenylenebis(3,1-benzoxazin-4-one) (compounds B1 to B3, comparative products 1 and 2) into a cylindrical glass container having a diameter of 30 mm and a height of 13 mm and using the TR-1800MK-II of Nippon Denshoku Co., Ltd. As for the measurement conditions, the reflection method (2 degree field of view, illuminant C) was employed.

$$YI = [100 - (1.28X - 1.06Z)]/Y$$

(the visual color difference is numerically calculated from three stimulus values X, Y and Z.)

Example 1: compound B1

(step 1: amidization)

25 g of anthranilic acid (manufactured by Tokyo Chemical Industry Co. , Ltd., HPLC purity: 99. 9 %, Na content: 60 ppm) was dissolved in 150 g of methyl isobutyl ketone (MIBK) in a 500 ml four-necked flask equipped with a thermometer, a stirrer, a reflux condenser, a dropping funnel, a nitrogen gas introduction unit and a gas lead-out unit connected to a hydrogen halide removing device. The temperature of the resulting solution was set to 55 to 60°C, and a solution prepared by dissolving 18 g of terephthalic acid dichloride (manufactured by Tokyo Chemical Industry Co., Ltd.) in 72 g of MIBK was added dropwise to the solution in 30 minutes. Thereafter, nitrogen was blown into the gas phase part of the reaction flask at a rate of 100 ml/min (6 liters/h) to carry out a reaction at 80 to 85°C for 12 hours. After the end of the reaction, the obtained solution was cooled to 30 °C or lower and filtered, and the obtained crystal was washed with 100 ml of MINK.

(step 2: dehydration)

The obtained crystal and 300 g of acetic anhydride were injected into a 500 ml four-necked flask equipped with a thermometer, a stirrer, a reflux condenser, a dropping funnel, a reflux liquid extraction port and a gas lead-out unit connected to an acidic gas removing device to carry out heating and reflux for 12 hours while about 10 g of the reflux liquid was distilled out each time in 1 hour. The temperature gradually rose and exceeded 130°C in the end. Thereafter, the solution was cooled to 30°C or lower and filtered.

This crystal and 120 g of methanol were injected into a 500 ml four-necked flask equipped with a thermometer, a stirrer, a reflux condenser, a dropping funnel and a gas lead-out unit connected to an acidic gas removing device and stirred at 50 to 60°C for 30 minutes. 120 g of ion exchange water was added dropwise to the resulting mixture, cooled to 30°C or lower and filtered, and the obtained crystal was washed with 120 g of methanol. The obtained crystal was dried at 60°C to obtain 29 g of slightly yellowish crystalline 2,2'-phenylenebis(3,1-benzoxazin-4-one) (compound B1).

The obtained compound B1 had an HPLC purity of 98.9 %, a content of the compound of the formula (7) of 0.05 wt%, a maximum peak in the measurement of light reflectance at 450 nm, a maximum light reflectance of 105 %, a metal sodium content of less than 1 ppm, and a YI value of -4.3. Its yield was 89.0 %.

Example 2: compound B2

(step 1: amidization)

25 g of anthranilic acid (manufactured by Fuso Chemical Co., Ltd., HPLC purity: 98.5 %, Na content: 590 ppm) was dissolved in 150 g of methyl isobutyl ketone (MIBK) in a 500 ml four-necked flask equipped with a thermometer, a stirrer, a reflux condenser, a dropping funnel, a nitrogen gas introduction unit and a gas lead-out unit connected to a hydrogen halide removing device. The temperature of the resulting solution was set to 55 to 60°C, and a solution prepared by dissolving 18 g of terephthalic acid dichloride (manufactured by Iharanikkei Chemical Industry Co., Ltd.) in 72 g of MIBK was added dropwise to the solution in 30 minutes. Thereafter, nitrogen was blown into the gas phase part of the reaction flask at a rate of 120 ml/min (7.2 liters/h) to carry out a reaction at 80 to 85°C for 10 hours. After the end of the reaction, the solution was cooled to 30°C or lower and filtered, and the obtained crystal was washed with 100 ml of MIBK.

(step 2: dehydration)

The obtained crystal and 300 g of acetic anhydride were injected into a 500 ml four-necked flask equipped with a thermometer, a stirrer, a reflux condenser, a dropping funnel, a reflux liquid extraction port and a gas lead-out unit connected to an acidic gas removing device to carry out heating and reflux for 12 hours while about 10 g of the reflux liquid was distilled out each time in 1 hour. The temperature gradually rose and exceeded 130°C in the end. Thereafter, the solution was cooled to 30°C or lower and filtered.

This crystal and 120 g of methanol were injected into a 500 ml four-necked flask equipped with a thermometer, a stirrer, a reflux condenser, a dropping funnel and a gas lead-out unit connected to an acidic gas removing device and stirred at 50 to 60°C for 30 minutes. 60 g of ion exchange water was added dropwise to the resulting mixture, cooled to 30°C or lower and filtered, and the obtained crystal was washed with 60 g of methanol. The obtained crystal was dried at 60°C to obtain 30.3 g of slightly yellowish crystalline 2,2'-phenylenebis(3,1-benzoxazin-4-one) (compound B2).

The obtained compound B2 had an HPLC purity of 98.6 %, a content of the compound of the formula (7) of 0.05 wt%, a maximum peak in the measurement of light reflectance at 450 nm, a maximum light reflectance of 103 %, a metal sodium content of 23 ppm and a YI value of -5.9. Its yield was 93.1 %.

Example 3: compound B3

(step 1: amidization)

25 g of anthranilic acid (manufactured by Mitsuboshi Chemical Industry Co., Ltd., HPLC purity: 99.2 %, Na content: 100 ppm) was dissolved in 150 g of methyl isobutyl ketone (MIBK) in a 500 ml four-necked flask equipped with a thermometer, a stirrer, a reflux condenser, a dropping funnel, a nitrogen gas introduction unit and a gas lead-out unit connected to a hydrogen halide removing device. The temperature of the resulting solution was set to 55 to 60°C, and a solution prepared by dissolving 18 g of terephthalic acid dichloride (manufactured by Ihara Nikkei Chemical Industry Co., Ltd.) in 72 g of MIBK was added dropwise to the solution in 30 minutes. Thereafter, nitrogen was blown into the gas phase part of the reaction flask at a rate of 33 ml/min (2 liters/h) to carry out a reaction at 80 to 85°C for 20 hours. After the end of the reaction, the solution was cooled to 30°C or lower and filtered, and the obtained crystal was washed with 100 ml of MIBK.

(step 2: dehydration)

The obtained crystal and 300 g of acetic anhydride were injected into a 500 ml four-necked flask equipped with a thermometer, a stirrer, a reflux condenser, a dropping funnel, a reflux liquid extraction port and a gas lead-out unit connected to an acidic gas removing device to carry out heating and reflux for 12 hours while about 10 g of the reflux liquid was distilled out each time in 1 hour. The temperature gradually rose and exceeded 130°C in the end. Thereafter, the solution was cooled to 30°C or lower and filtered.

This crystal and 120 g of methanol were injected into a 500 ml four-necked flask equipped with a thermometer, a stirrer, a reflux condenser, a dropping funnel and a gas lead-out unit connected to an acidic gas removing device and stirred at 50 to 60°C for 30 minutes. 120 g of ion exchange water was added dropwise to the resulting mixture, cooled to 30°C or lower and filtered, and the obtained crystal was washed with 120 g of methanol. The obtained crystal was dried at 60°C to obtain 29 g of slightly yellowish crystalline 2,2'-phenylenebis(3,1-benzoxazin-4-one) (compound B3).

The obtained compound B3 had an HPLC purity of 99.4 %, a content of the compound of the formula (7) of 0.08 wt%, a maximum peak in the measurement of light reflectance at 450 nm, a maximum light reflectance of 101 %, a metal sodium content of less than 1 ppm and a YI value of 0.5. Its yield was 89.6 %.

Comparative Example 1: comparative product 1

(step 1: amidization)

25 g of anthranilic acid (manufactured by Mitsuboshi Chemical Co., Ltd., HPLC purity: 99.2 %, Na content: 100 ppm) and 20.9 g of sodium carbonate were dissolved in 446 g of water in a 1,000 ml four-necked flask equipped with a thermometer, a stirrer, a reflux condenser, a dropping funnel, a nitrogen gas introduction unit and a gas lead-out unit connected to a hydrogen halide removing device to prepare an alkaline aqueous solution, and an organic solvent solution prepared by dissolving 18 g of terephthalic acid dichloride (manufactured by Tokyo Chemical Industry Co., Ltd.) in 107 g of acetone was added dropwise to this alkaline aqueous solution at 20 to 30°C under agitation and then mixed with the solution at room temperature for 2 hours and further under the reflux of acetone for 1 hour to carry out a reaction. Thereafter, concentrated hydrochloric acid was added to make the reaction system acidic, and the reaction solution was filtered and dried to obtain 34.1 g of N,N'-bis(O-carboxyphenyl)terephthalamide.

(step 2: dehydration)

The obtained crystal and 178.6 g of acetic anhydride were injected into a 1,000 ml four-necked flask equipped with a thermometer, a stirrer, a reflux condenser, a dropping funnel, a reflux liquid extraction port and a gas lead-out unit connected to an acidic gas removing device to carry out a reaction under the reflux of acetic anhydride for 2 hours. The reaction product was cooled, filtered and dried to obtain 27.7 g of slightly yellowish crystalline 2,2'-phenylenebis(3,1-benzoxazin-4-one) (comparative product 1).

The obtained comparative product 1 had an HPLC purity of 99.9 %, a content of the compound of the formula 7) of 0.04 wt%, a maximum peak in the measurement of light reflectance at 450 nm., a maximum light reflectance of 80 %, a metal sodium content of 200 ppm and a YI value of 13.0. Its yield was 89.0 %.

Comparative Example 2: comparative product 2

Commercially available isatoic acid (manufactured by BASF AG, HPLC purity: 97.5 %, Na content: 2000 ppm) was dissolved in dimethyl formamide (DMF) at 60°C in a 500 ml four-necked flask equipped with a thermometer, a stirrer, a reflux condenser, a dropping funnel, a nitrogen gas introduction unit and a gas lead-out unit connected to a hydrogen halide removing device to be recrystallized. The resulting mixture was left to be cooled, and the crystal was filtered and dried to obtain purified isatoic anhydride.

Thereafter, 25 g of the purified and dried isatoic anhydride was dissolved in 250 g of dry pyridine at a temperature of about 60°C. 15.8 g of terephthaloyl dichloride manufactured by E.I. du Pont de Nemours and Company was gradually added to the isatoic anhydride mixture under agitation while it was slightly cooled to maintain the temperature of the solution. The precipitate was filtered, cleaned and dried to obtain 43.86 g of slightly yellowish crystalline 2,2'-phenylenebis(3,1-benzoxazin-4-one) (comparative product 2).

The obtained comparative product 2 had an HPLC purity of 96.7 %, a content of the compound of the formula (7) of 0.26 wt%, a maximum peak in the measurement of light reflectance at 450 nm, a maximum light reflectance of 91 %, a metal sodium content of 13 ppm and a YI value of -5.8. Its yield was 85.0 %.

<resin composition>

The characteristic properties of a molded product were measured by the following methods.

(1) maximum peak of reflectance

A 50 mm square plate having a thickness of 2 mm was produced by using a metal mold having a cavity surface with an arithmetic average roughness (Ra) of 0.03 μm and an injection molding machine having a maximum clamping force of 150 tons at a cylinder temperature of 320°C, a mold temperature of 80°C and a molding cycle of 60 seconds and evaluated. The maximum peak of reflectance of the obtained molded article was measured with the TR-1800MK-II of Nippon Denshoku Co., Ltd. by placing a white standard plate on the obtained molded article.

(2) total light transmittance

The total light transmittance of the molded article obtained in (1) was measured with the PC-305SIII/V of Hirayama Manufacturing Corporation (conditions; illuminant C, 2 degree field of view).

(3) color of molded article

The YI of the molded article obtained in (1) was measured from the following equation by using the TR-1800MK-II of Tokyo Denshoku Co., Ltd. (conditions; illuminant C, 2 degree field of view).

$$YI = [100 - (1.28X - 1.06Z)]/Y$$

(the visual color difference was numerically calculated from three stimulus values X, Y and Z.)

(6) moist heat resistance

The difference between haze after the molded article obtained in (1) was treated at 120°C and a relative humidity of 95 % for 24 hours and haze before the above treatment was measured with the PC-305SIII/V of Hirayama Manufacturing Corporation and expressed as Δhaze.

$$\Delta haze = haze\ after\ treatment - haze\ before\ treatment$$

(7) weatherability (weatherability promotion test)

The difference between haze after 1,000 hours of a treatment, each cycle of which consisted of spraying water for 18 minutes and not spraying water for 102 minutes, measured with a sunshine weather meter (WEL-SUN-HC of Suga Test Instruments Co., Ltd..) at a black panel temperature of 63°C and a humidity of 50 %, and haze before the above treatment was expressed as Δhaze.

$$\Delta haze = haze\ after\ treatment - haze\ before\ treatment$$

<Examples 4 to 7 and Comparative Examples 3 to 6>

Additives shown in Table 2 were added in amounts shown in Table 2 to 100 parts by weight of an aromatic polycarbonate obtained from bisphenol A and phosgene by the interfacial polycondensation process, and the resulting mixture was blended with 0.0005 part by weight of an anthraquinone-based dye (MACROLEX Violet B of Bayer AG) by means of a blender and melt kneaded with the dye by means of a vented double-screw extruder to obtain a pellet.

After a pre-mixture of the additives and the aromatic polycarbonate was prepared to ensure that the concentrations of the additives to be added to the aromatic polycarbonate were 10 to 100 times the amounts thereof, it was wholly mixed by means of a blender. The TEX30α vented double-screw extruder (completely mated, same-direction rotation, two screws) of The Japan Steel Works, Ltd. was used. One kneading zone was existent before the vent port. As for extrusion conditions, the delivery rate was 25 kg/h, the screw revolution was 140 rpm, the vacuum degree of the

vent was 3 kPa, and the extrusion temperature from the first supply port to the dice was 280°C.

The obtained pellet was dried at 120°C for 5 hours with a hot air circulation drier and molded into a 2 mm-thick 50 mm square plate at a cylinder temperature of 320°C and a mold temperature of 80°C with an injection molding machine. The SG260M-HP injection molding machine of Sumitomo Heavy Industries, Ltd. was used. The evaluation results of the obtained molded plate are shown in Table 2. The components shown in Table 2 denote the following.

(component A: aromatic polycarbonate)

PC: aromatic polycarbonate powder having a viscosity average molecular weight of 22,400 obtained from bisphenol A and phosgene by interfacial polycondensation process (Panlite L-1225WP of Teijin Chemicals Ltd.)

(component C: stabilizer)

C1: phosphite-based stabilizer (Irgafos168 of Ciba Specialty Chemicals Holdings Inc.)

C2: hindered phenol-based stabilizer (Irganox1076 of Ciba Specialty Chemicals Holdings Inc.)

(component D: anthraquinone-based dye)

D1: MACROLEX Violet B of Bayer AG

(other components)

release agent: full ester having a molecular weight of 925 of pentaerythritol and an aliphatic carboxylic acid (Rikestar EW-400 of Riken Vitamin Co., Ltd..)

Table 1

| | Example No. | - | Ex. 1 | Ex. 2 | Ex. 3 | C.Ex.1 | C.Ex.2 |
|---|---|---|---|---|---|---|---|
| | Compound No. | - | B1 | B2 | B3 | Comparative Product 1 | Comparative Product 2 |
| Bisbenzoxazinone compound | YI | Illuminant C, 2 degree | -4.3 | -5.9 | 0.5 | 13.0 | -5.8 |
| | Na | ppm | Less than 1 | 23 | Less than 1 | 200 | 13 |
| | Particle size distribution | Less than 10μm | 23.5 | 16.6 | 21.3 | 21.9 | 35.9 |
| | wt% | 10 to 30μm | 62.0 | 72.1 | 66.2 | 56.6 | 57.3 |
| | wt% | More than 30μm | 14.5 | 11.3 | 12.5 | 21.5 | 6.8 |
| | Arithmetic average diameter | μm | 18.8 | 16.2 | 19.2 | 21.9 | 13.9 |
| | Maximum light reflectance of its powder | % | 105 | 103 | 101 | 80 | 91 |
| | purity | % | 98.9 | 98.6 | 99.4 | 99.9 | 96.7 |
| | Content of compound of formula (7) | wt% | 0.05 | 0.05 | 0.08 | 0.04 | 0.26 |
| Raw material anthranilic acid | Purity | % | 99.9 | 98.5 | 99.2 | 99.2 | - |
| Raw material isatoic acid | Purity | % | - | - | - | - | 97.5 |
| Ex.: Example C.Ex.: Comparative Example | | | | | | | |

Table 2

| | | Unit | Ex. 4 | Ex. 5 | Ex. 6 | Ex. 7 |
|---|---|---|---|---|---|---|
| PC (Component A) | | wt% | 100 | 100 | 100 | 100 |
| Bisbenzoxazinone (component B) | Example 1 (Compound B1) | wt% | 0.3 | 3 | | |
| | Example 2 (Compound B2) | wt% | | | 0.3 | |
| | Example 3 (Compound B3) | wt% | | | | 0.3 |
| | Comparative Example 1 (Comparative product 1) | wt% | | | | |
| | Comparative Example 2 (Comparative product 2) | wt% | | | | |
| Anthraquinone-based dye (component D) | D1 | wt% | 0.6 | 0.6 | 0.6 | 0.6 |
| Release agent | EW-400 | wt% | 0.1 | 0.1 | 0.1 | 0.1 |
| Stabilizer (component C) | C1 | wt% | 0.03 | 0.03 | 0.03 | 0.03 |
| | C2 | wt% | 0.05 | 0.05 | 0.05 | 0.05 |
| Optical properties | total light transmittance | % | 89.6 | 89.1 | 89.0 | 89.6 |
| Optical properties | maximum peak of light reflectance | — | 67.5 | 70.3 | 67.0 | 67.0 |
| Color | YI value (molded article 2mmt) | — | 0.6 | 1.3 | 0.7 | 0.9 |
| Moist heat resistance | Δhaze (before and after moist heat test) | — | 0.3 | 0.9 | 0.5 | 0.2 |
| Weatherability | Δhaze (before and after weatherbility promotion test) | — | 0.5 | 0.4 | 0.6 | 0.3 |

Ex.: Example

*1: Tokyo Denshoku Co., Ltd.(TC-1800MK-I)

*2: 120°C X 95%RH X 24hr

*3: S.W.O.M X 1000 hr

Table 2 (continued)

| | | Unit | C.Ex.3 | C.Ex.4 | C.Ex.5 | C.Ex.6 |
|---|---|---|---|---|---|---|
| PC (Component A) | | wt% | 100 | 100 | 100 | 100 |
| Bisbenzoxazinone (component B) | Example 1 (Compound B1) | wt% | | | | |
| | Example 2 (Compound B2) | wt% | 6 | | | 0.005 |
| | Example 3 (Compound B3) | wt% | | | | |
| | Comparative Example 1 (Comparative product 1) | wt% | | 0.3 | | |
| | Comparative Example 2 (Comparative product 2) | wt% | | | 0.3 | |
| Anthraquinone-based dye (component D) | D1 | wt% | 0.6 | 0.6 | 0.6 | 0.6 |
| Release agent | EW-400 | wt% | 0.1 | 0.1 | 0.1 | 0.1 |
| Stabilizer (component C) | C1 | wt% | 0.03 | 0.03 | 0.03 | 0.03 |
| | C2 | wt% | 0.05 | 0.05 | 0.05 | 0.05 |
| Optical properties | total light transmittance | % | 88.5 | 89.7 | 89.5 | 89.7 |
| Optical properties | maximum peak of light reflectance | — | 75.8 | 65.0 | 67.0 | 69.2 |
| Color | YI value (molded article 2mmt) | — | 2.1 | 1.0 | 0.9 | 1.0 |
| Moist heat resistance | Δhaze (before and after moist heat test) | — | 2.5 | 2.5 | 0.8 | 1.2 |
| Weatherability | Δhaze (before and after weatherbility promotion test) | — | 0.4 | 0.8 | 1.3 | 1.0 |

C.Ex.: Comparative Example

*1: Tokyo Denshoku Co., Ltd.(TC-1800MK-I)

*2: 120°C X 95%RH X 24hr

*3: S.W.O.M X 1000 hr

Effect of the Invention

The bisbenzoxazinone compound produced according to the present invention has excellent ultraviolet absorption ability and can improve the weatherability of a polycarbonate resin. Since the bisbenzoxazinone compound has high purity and a low content of metal sodium, it can prevent the hydrolysis under moist heat of a polycarbonate resin by an alkali metal. Since the bisbenzoxazinone compound generates fluorescence and has a high maximum light reflectance and a satisfactory YI value, it can improve the color of a polycarbonate resin.

According to the production method of the present invention, as an alkali metal compound such as sodium hydroxide or sodium carbonate is not used to remove hydrogen halide by-produced when the amide compound (3) is produced as shown in the above reaction formula, the obtained bisbenzoxazinone compound does not substantially contain an alkali metal salt and has high purity. According to the production method of the present invention, the bisbenzoxazinone compound can be produced at a high yield.

Industrial Applicability

The molded article can be used as a car transparent member.

## Claims

1. A method of producing a bisbenzoxazinone compound represented by the following formula (4):

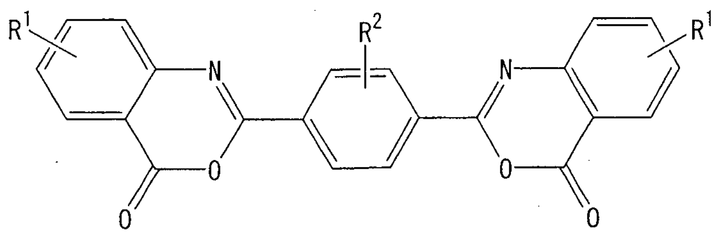

(4)

($R^1$ and $R^2$ are each independently a hydrogen atom, hydroxyl group, alkyl group having 1 to 3 carbon atoms, alkoxy group having 1 to 3 carbon atoms, acyl group having 1 to 3 carbon atoms, acyloxy group having 1 to 3 carbon atoms, alkoxycarbonyl group having 1 to 3 carbon atoms, halogen atom, nitro group or carboxyl group), and having

(i) a purity of 98 % or more,
(ii) a content of a compound represented by the following formula (7) of less than 0.15 wt%:

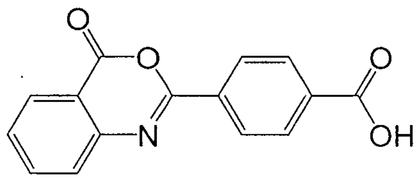

(7)

(iii) a maximum peak in the measurement of the light reflectance at a visible range of its powder at 380 to 480 nm and a maximum light reflectance of 100 to 120 %,
(iv) a metal sodium content of less than 50 ppm and
(v) a YI value of -10 to 10, comprising the steps of:

(Step 1) reacting an anthranilic acid derivative represented by the following formula (1) with an aromatic dicarboxylic acid dihalide represented by the following formula (2) in an organic solvent in an inert gas stream to produce an amide compound represented by the following formula (3), wherein the amount of

the inert gas is 1 to 50 liters/hr based on 1 mole of the anthralinic acid derivative:

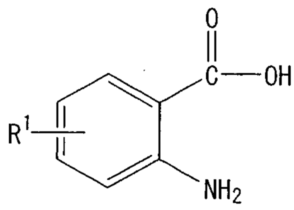

(1)

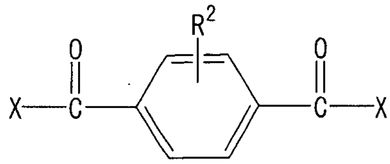

(2)

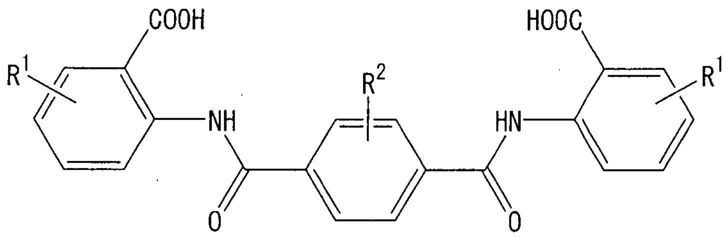

(3)

($R^1$ and $R^2$ are as defined above, and X is a halogen atom); and
(Step 2) reacting the obtained amide compound represented by the formula (3) with a dehydrating agent.

2. The production method according to claim 1, wherein the organic solvent is at least one selected from the group consisting of a ketone, an aromatic hydrocarbon, a halogenated hydrocarbon and an ether.

3. The production method according to claim 2, wherein the inert gas is nitrogen.

4. The production method according to claim 2, wherein the dehydrating agent is acetic anhydride.

5. The production method according to claim 2, wherein, in the formulas (1) to (4), $R^1$ is a hydrogen atom, $R^2$ is a hydrogen atom, and X is a chlorine atom.

## Patentansprüche

1. Verfahren zur Herstellung einer Bisbenzoxazinon-Verbindung der folgenden Formel (4):

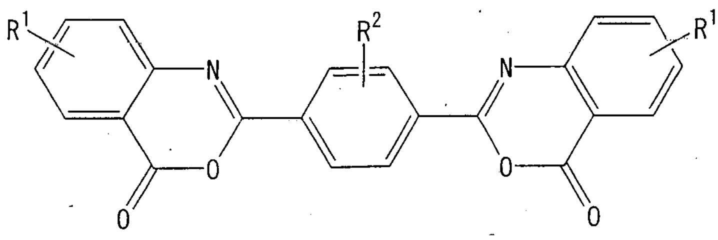

(4)

($R^1$ und $R^2$ sind jeweils unabhängig ein Wasserstoffatom, eine Hydroxylgruppe, eine Alkylgruppe mit 1 bis 3 Kohlenstoffatomen, eine Alkoxygruppe mit 1 bis 3 Kohlenstoffatomen, eine Acylgruppe mit 1 bis 3 Kohlenstoffatomen, eine Acyloxygruppe mit 1 bis 3 Kohlenstoffatomen, eine Alkoxycarbonylgruppe mit 1 bis 3 Kohlenstoffatomen, ein Halogenatom, eine Nitrogruppe oder eine Carboxylgruppe),
und mit

(i) einer Reinheit von 98% oder mehr,
(ii) einem Gehalt an einer Verbindung der folgenden Formel (7) von weniger als 0,15 Gew.-%:

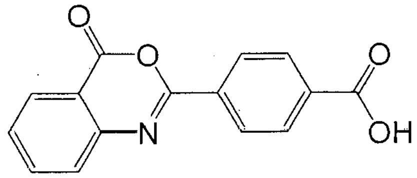

(7)

(iii) einem Maximumpeak bei der Messung des Lichtreflexionsvermögens in einem sichtbaren Bereich von seinem Pulver bei 380 bis 480 nm und einem maximalen Lichtreflexionsvermögen von 100 bis 120%,
(iv) einem Metallnatriumgehalt von weniger als 50 ppm und
(v) einem YI-Wert von -10 bis 10, umfassend die Stufen:

(Stufe 1) das Umsetzen eines Anthranilsäurederivats der folgenden Formel (1) mit einem aromatischen Dicarbonsäuredihalogenid der folgenden Formel (2) in einem organischen Lösungsmittel in einem inerten Gasstrom, um eine Amidverbindung der folgenden Formel (3) herzustellen, wobei die Menge des inerten Gases 1 bis 50 Liter/Std, bezogen auf 1 Mol des Anthranilsäurederivats, beträgt:

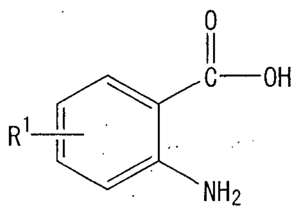

(1)

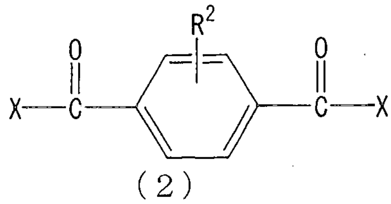

(2)

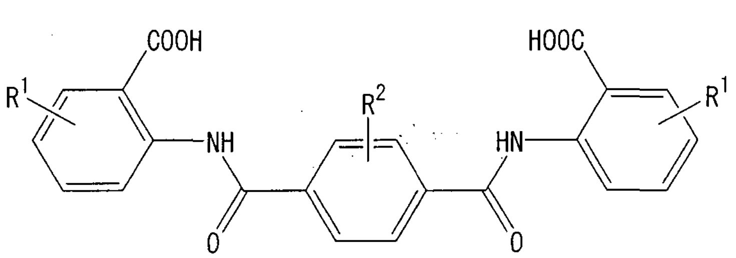

(3)

($R^1$ und $R^2$ sind wie oben definiert und X ist ein Halogenatom) ; und

(Stufe 2) das Umsetzen der erhaltenen Amidverbindung der Formel (3) mit einem Entwässerungsmittel.

2. Herstellungsverfahren nach Anspruch 1, wobei das organische Lösungsmittel mindestens eines, ausgewählt aus der Gruppe, bestehend aus einem Keton, einem aromatischen Kohlenwasserstoff, einem halogenierten Kohlenwasserstoff und einem Ether, ist.

3. Herstellungsverfahren nach Anspruch 2, wobei das inerte Gas Stickstoff ist.

4. Herstellungsverfahren nach Anspruch 2, wobei das Entwässerungsmittel Essigsäureanhydrid ist.

5. Herstellungsverfahren nach Anspruch 2, wobei in den Formeln (1) bis (4) $R^1$ ein Wasserstoffatom ist, $R^2$ ein Wasserstoffatom ist und X ein Chloratom ist.

## Revendications

1. Procédé de production d'un composé bisbenzoxazinone représenté par la formule (4) suivante :

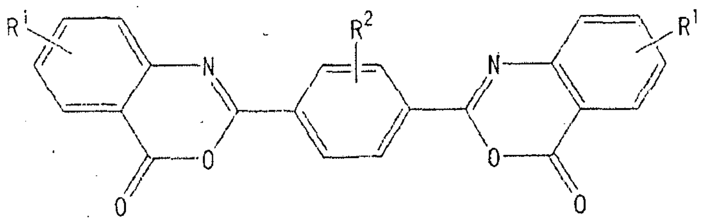

(4)

($R^1$ et $R^2$ sont chacun indépendamment un atome d'hydrogène, un groupe hydroxyle, un groupe alkyle ayant de 1 à 3 atomes de carbone, un groupe alcoxy ayant de 1 à 3 atomes de carbone, un groupe acyle ayant de 1 à 3 atomes de carbone, un groupe acyloxy ayant de 1 à 3 atomes de carbone, un groupe alcoxycarbonyle ayant de 1 à 3 atomes de carbone, un atome d'halogène, un groupe nitro ou un groupe carboxyle),
et ayant

(i) une pureté de 89 % ou plus,
(ii) une teneur en un composé représenté par la formule suivante (7) inférieure à 0,15 % en poids :

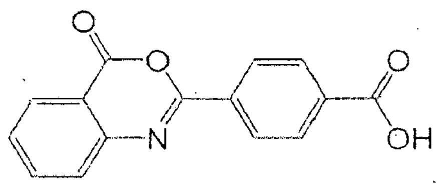

(7)

(iii) un pic maximal dans la mesure du facteur de réflexion de la lumière dans une plage visible de sa poudre à 380 à 480 nm et un facteur de réflexion de la lumière maximal de 100 à 120 %,
(iv) une teneur en sodium métallique inférieure à 50 ppm et
(v) une valeur YI de -10 à 10, comprenant les étapes suivantes :

(étape 1) mise en réaction d'un dérivé d'acide anthranilique représenté par la formule (1) suivante avec un dihalogénure d'acide dicarboxylique aromatique représenté par la formule (2) suivante dans un solvant organique dans un courant de gaz inerte pour produire un composé amide représenté par la formule (3) suivante, dans lequel la quantité de gaz inerte est de 1 à 50 L/h sur la base de 1 mole du dérivé d'acide anthranilique :

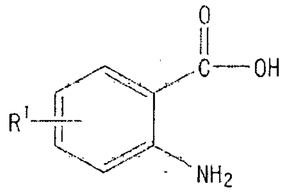

(1)

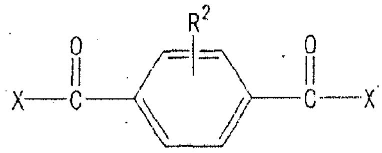

(2)

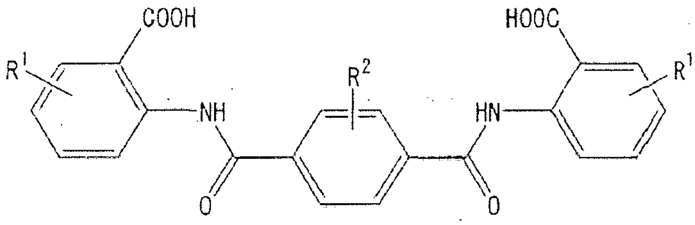

(3)

($R^1$ et $R^2$ sont tels que définis ci-dessus, et X est un atome d'halogène) ;
et
(étape 2) mise en réaction du composé amide obtenu représenté par la formule (3) avec un agent de déshydratation.

2. Procédé de production selon la revendication 1, dans lequel le solvant organique est au moins un solvant sélectionné dans le groupe constitué d'une cétone, d'un hydrocarbure aromatique, d'un hydrocarbure halogéné et d'un éther.

3. Procédé de production selon la revendication 2, dans lequel le gaz inerte est l'azote.

4. Procédé de production selon la revendication 2, dans lequel l'agent de déshydratation est l'anhydride acétique.

5. Procédé de production selon la revendication 2, dans lequel, dans les formules (1) à (4), $R^1$ est un atome d'hydrogène, $R^2$ est un atome d'hydrogène, et X est un atome de chlore.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 58194854 A
- JP 61291575 A
- JP 2003155468 A
- WO 2003035735 A